(19) Europäisches Patentamt
European Patent Office
Office européen des brevets

(11) Publication number: **0 354 847**
**A2**

(12) # EUROPEAN PATENT APPLICATION

(21) Application number: **89402254.0**

(22) Date of filing: **09.08.89**

(51) Int. Cl.[5]: **G 01 N 33/532**
G 01 N 33/58, G 01 N 33/533

(30) Priority: **11.08.88 CA 574483**

(43) Date of publication of application:
**14.02.90 Bulletin 90/07**

(84) Designated Contracting States:
**AT BE CH DE ES FR GB IT LI NL SE**

(71) Applicant: **CYBERFLUOR INC.**
**179 John Street 4th Floor**
**Toronto Ontario M5T 1X4 (CA)**

(72) Inventor: **Diamandis, Eleftherios P.**
**179 John Street 4th floor**
**Toronto Ontario M5T 1X4 (CA)**

**Morton, Robert C.**
**601 Geneva Park Drive**
**Burlington Ontario L7N 3C2 (CA)**

(74) Representative: **Gillard, Marie-Louise et al**
**Cabinet Beau de Loménie 55, Rue d'Amsterdam**
**F-75008 Paris (FR)**

(54) **Multiple fluorescence labelling with europium chelators.**

(57) A conjugate for use in a labelling system comprises avidin or streptavidin linked to a carrier particle, having more than fifteen amino groups on its surface, which are individually capable of being labelled with an operable label. The carrier particle is capable of being linked to the avidin or streptavidin to form the conjugate. The conjugate is particularly useful in forming a fluorescent reagent system. The carrier particle is labelled with a plurality of ligand moieties forming with a lanthanide metal ion, fluorescent chelates attached to the plurality of amino groups to provide at least fifteen labels. The fluorescent reagent system, when excited to fluoresce, emits fluorescent radiation with an intensity amplified compared to intensity of fluorescent radiation emitted by the plurality of fluorescent lanthanide metal ion chelate moieties in a solution whereby the fluorescent reagent is void of any quenching effects due to multiple labelling in the fluorescent reagent system. The fluorescent labelling system is particularly useful in binding assays.

EP 0 354 847 A2

**Description**

# MULTIPLE FLUORESCENCE LABELLING WITH EUROPIUM CHELATORS

## FIELD OF THE INVENTION

This invention relates to multiple labelling in an assay with a suitable labelling which avoids the known effects of quenching due to such multiple labelling.

## BACKGROUND OF THE INVENTION

Fluorescence labelling with conventional probes like fluorescein, rhodamine, dansyl chloride, fluorescamine and the newer labels Texas Red and phycobiliproteins is widely used for analytical and non-analytical applications. Fluorescent labels are now used extensively in the field of fluorescence immunoassay as alternatives to the radioactive labels. However, the limit of detection is restricted to the micromolar and nanomolar range mainly because of the high background fluorescence readings encountered in the measurements and the limited number of labels which can be incorporated into the immunoreactants.

In principle, the sensitivity of the fluoroimmunoassay could be improved by attaching many fluorescent groups to the labelled reagent. This could be achieved directly (for macromolecular reagents) or indirectly through conjugation of the reagent to a carrier macromolecule labelled with fluorescent molecules. Unfortunately, these approaches do not work because the increased tagging level soon produces concentration quenching which reduces first the quantum efficiency and then the total emission. These problems are disclosed in more detail, Chen R.F., Arch. Biochem. Biophys. 1969; 133:263-276; Hassan M, Landon J, Smith D.S., FEBS Lett. 197, 103:339-341; Hirschfeld T., Appl. Optics. 1976; 15:3135-9. For example, when a macromolecule was labelled with 32 fluorescein molecules, an emission intensity equivalent to only two free fluorescein molecules was obtained as noted in the above reference by Hirschfeld.

More recently, a bulking agent that is covalently bound to the antigen has been used to disperse the fluorescein labels and minimize concentration quenching. With this technique, a maximum of 8.8 fluorescein molecules have been incorporated yielding a fluorescence intensity equivalent to that of 7.7 fluorescein molecules as disclosed in Rowley, G.L., Henriksson, T., Louie, A, et al. Clin. Chem. 1987; 33: 1536.

Alternatively, multiple labelling can be used with fluorophores which are not subject to concentration quenching. Multi-umbelliferone substituted polylysine labels have been used successfully in steroid fluoroimmunoassays as reported in Exley, D., Ekeke, G.I., J. Steroid Biochem. 1981, 14:1297-302. The umbelliferone fluorophor showed no concentration quenching at the level of 1 fluor/10 lysine residues.

Hirschfeld (supra) proposed an elegant method to overcome the problem of concentration quenching of fluorescein. He demonstrated that when the sample is illuminated to complete photochemical bleaching using a powerful laser, each fluorophor emits the same maximum number of photons. He thus achieved a linear increase of the signal with the number of labels used, regardless of concentration quenching. Using this technique, which is nevertheless not practical because of a complex and costly instrumentation, he was able to detect single antibody molecules labelled with multi-fluorescein-substituted polyethylenemine as noted in his other paper Hirschfeld, T., Appl. Optics. 1976; 15:2965-6.

According to this invention, a multiple labelling system is provided which avoids the quenching effect normally experienced and instead provides an enhanced signal from the excited label.

## SUMMARY OF THE INVENTION

According to an aspect of the invention, a conjugate for use in a labelling system comprises avidin or streptavidin linked to a carrier particle having more than fifteen amino groups on its surface which are individually capable of being labelled with an operable label. The carrier particle is capable of being linked to avidin or streptavidin to form the conjugate.

According to another aspect of the invention, a fluorescent reagent system for use in an assay comprises the conjugate having the avidin or streptavidin suitably linked by biotin to an assay component. The carrier particle is labelled with a plurality of moieties of a ligand attached to the plurality of amine groups to provide at least fifteen labels. Each of the labels in the presence of a selected lanthanide metal ion forms a plurality of fluorescent chelates. The fluorescent reagent system when excited to fluoresce emits fluorescent radiation with an intensity amplified compared to an intensity of fluorescent radiation emitted by the plurality of the fluorescent lanthanide metal ion chelate moieties in a solution. As a result, the fluorescent reagent is void of any quenching effects due to a multiple labelling system in a fluorescent reagent system.

According to another aspect of the invention in a binding assay, the use of a binder having a plurality of the above conjugates chemically linked thereto by a corresponding plurality of linking components. The carrier particle is labelled with a plurality of labels comprising moieties of a ligand attached to a respective amine group. Each of the moieties has stably retained thereon a selected lanthanide metal ion to provide fluorescent chelates. Each of the linking components comprises a biotin molecule chemically linked to the binder and bound to the avidin or streptavidin.

BRIEF DESCRIPTION OF THE DRAWINGS

Various aspects of the multiple labelling system according to this invention, are exemplified in the following drawings wherein

Figure 1 is a schematic representation of the BCPDA labelled reagents and their utilization on an AFP assay. In the figure, ℨ = solid phase, · = BCPDA (4,7-bis (chlorosulfophenyl)-1,10-phenathroline-2,9-dicarboxylic acid), AFP = α-fetoprotein, B = biotin, SA = streptavidin, and TG = thyroglobulin;

Figure 2 shows the elution behavior of the conjugate according to an aspect of this invention from an Ultrogel A 34 column;

Figure 3 is the fluorescent quantitation of the binding of streptavidin to biotinylated antibody coated on microtitration plates;

Figure 4 shows the calibration curves for the α-fetoprotein assay with directly labelled antibody "A" or biotinylated antibody and directly labelled streptavidin "B" or streptavidin conjugated to labelled thyroglobulin "C";

Figure 5 is a schematic representation of an alternative embodiment for BCPDA labelled reagents in forming macromolecular complexes. In the Figure, · = BCPDA; TG = thyroglobulin; SA = streptavidin;

Figure 6 is a graph representing saturation of BCPDA by europium;

Figure 7 is a calabration curve for the prolactin assay;

Figure 8 is a plot of the relative stability of the macromolecular complex in the presence or absence or europium;

Figure 9 is a graph plotting the elution behavior of assay-TG (BCPDA) 150 conjugate from a Sephacryl S-400™ column; and

Figure 10 is the elution behavior of the SBMC in the presence of excess ETDA from a Sephadex G-25™ column.

DETAILED DESCRIPTION OF THE PREFERRED EMBODIMENTS

Assays based on fluorescence have gained considerable prominence in recent years compared to radioimmunoassays. Theoretically, fluorescent label systems particularly involving europium chelates are generally understood to be more sensitive than $^{125}I$ normally used in radioimmunoassays. Alternative labels which include fluorescent labels are therefore gaining prominence in the field of assays. Other than fluorescent labels, other components used in providing labels include chemiluminescent probes such as acridinium esters and luminol derivatives, enzyme probes such as horseradish peroxidase, β-galactosidase and alkaline phosphatase as well as the metal ion probes such as the lanthanide metal ions which include europium.

In the field of fluorescent europium chelates as labels, there are two general assay configurations. The first approach is to use $Eu^{3+}$ bound to antibody molecules via a bridge (chelator) such as an ethylenediaminetetraacedic acid (EDTA) derivative. This procedure is identified in Lovgren, T., Hemmila, I., Petterson, K., Halonen, P., In: Collins WP, Ed. Alternative Immunoassays. pp. 203-217. New York: J. Wiley, 1985. After the immunological reaction is complete, $Eu^{3+}$ is dissociated from EDTA by lowering pH. Then the $Eu^{3+}$ is complexed with appropriate ligands such as naphthoyltrifluoroacetone (NTA) and trioctyl phosphine oxide (TOPO). The fluorescence is then measured in a time resolved mode in solution. This two- step procedure is necessary because the EDTA-$Eu^{3+}$ complex is not in and of itself fluorescent. This configuration therefore suffers from a number of disadvantages, the most prominent of which is its vulnerability to extraneous $Eu^{3+}$ contamination. However, the system still offers a very sensitive label in that the optimized complexation of $Eu^{3+}$ and the formation of the $Eu^{3+}$-$(NTA)_3$ $(TOPO)_2$ complex provides a very low detection limit in the range of approximately $10^{-13}$ mol/l of $Eu^{3+}$. This aspect is confirmed by Hemmilla, I., Dakubu, S., Mukkala, V.M., Siitari, H., Lovgren, T., Anal. Biochem. 1984; 137:375-343.

The second approach in using a europium chelator as the label is to introduce excess $Eu^{3+}$ to form the fluorescent complex. A suitable chelator for this purpose has been synthesized and identified as a derivative of 1,10-phenanthroline-2,9-carboxylic acid which in a specific embodiment is 4,7-bis (chlorosulfophenyl)-1,10-phenathroline-2,9-dicarboxylic acid, hereinafter referred to as BCPDA. The synthesis of this compound is disclosed in Evangelista RA, Pollak A, Allore B, Templeton, EF, Morton RC, Diamandis EP. Clin. Biochem. 1988; 21: 173-178. Further reference to this chelator compound can be found in applicant's co-pending Canadian patent application 488,513 filed August 12, 1985 and an earlier European application 85305477.3 filed July 31, 1985. Such ligand in forming a fluorescent metal ion chelate may be applied to the immunoassay of number of biological compounds. In principal, this assay configuration is advantageous because $Eu^{3+}$ contamination problem is eliminated and fluorescence can be measured directly on the solid phase. This approach, however, suffers from one disadvantage, because the $Eu^{3+}$ is used in excess. Only the 1:1 BCPDA:$Eu^{3+}$ complex is formed and it has a lower fluorescence yield than complexes of the form $Eu^{3+}$-$(BCPDA)_n$, where $n = 2$ or 3. Thus BCPDA can be detected down to approximately $10^{-11}$ mole/L which is two orders of magnitude higher than the detection limit of measuring $Eu^{3+}$ with excess chelators.

The preferred moieties for the 1,10-phenanthroline-2,9-dicarboxylic acid ligand compounds are selected from the group consisting of compounds represented by formula I:

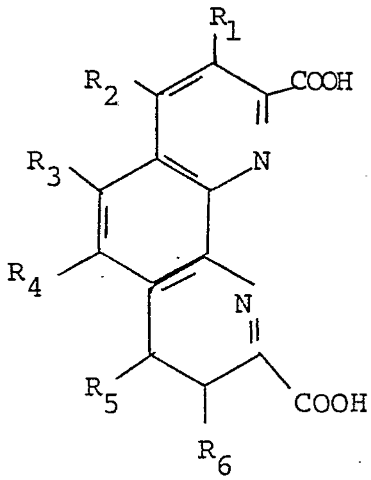

(I)

wherein:
each $R_1$ to $R_6$ group is independently hydrogen, $X(R_7)_n$ or $R_8$- and X is $-SO_3^-M^+$ wherein M is metal ion or is a functional group which couples covalently with proteins or a group readily convertible to functional group which couples covalently with proteins, $R_7$ is a divalent aliphatic residue having 1 to 12 carbons, or a divalent carboxylic or heterocyclic residue having 3 to 12 carbons, and n is 0 or 1, and;
wherein
$R_8$ is an aliphatic group having 1 to 12 carbons, or a carboxylic or heterocyclic group having 3 to 12 carbons or one or more pairs of adjacent $R_1$ to $R_6$ groups form together with the carbons to which they are substituents (a) a carboxylic or heterocyclic ring containing 3 to 12 carbons, or (b) an X-substituted carboxylic or heterocyclic ring of the general formula Ia:

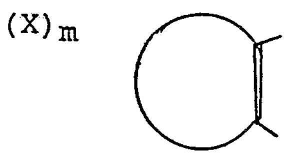

Ia

wherein

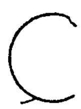

is a divalent carboxylic or residue having 1 to 12 carbons, X has the signification given above, and m is an integer from 1 to 4, or (c) an orthoquinone linkage:

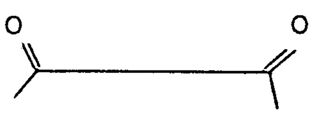

with the proviso that at least one of $R_1$ to $R_6$ is $X(R_7)_n$, wherein X, $R_7$, and n have the significations given above, or at least one pair of adjacent $R_1$ to $R_6$ groups form a ring of the formula Ia given above or an orthoquinone linkage, and trihalomethyl forms, salts, esters and said halides thereof which are readily hydrolyzed to form the acid of formula I.

As noted in applicant's co-pending application, it has been found that moieties of the 4,7-diphenyl-1,10-phenanthroline-2,9-dicarboxylic acid are particularly useful in forming highly stable fluorescent chelates with lanthanide metal ions, preferably with the europium metal ion such that the metal ion remains stably attached to solid phase and allows the assay to be conducted by measurement of fluorescence of the chelate bound to the solid phase. The preferred ligand is 4,7-bis(chlorosulfophenyl)-1,10 phenanthroline 2,9 dicarboxylic acid.

The invention is, however, by no means limited to use of the above mentioned ligand compounds. The suitability of other fluorescent chelates for use in the present method can be determined by trial and experiment having regard to the above principals and detailed disclosure. Immunoreactive bodies, especially antigens and antibodies which are often desired to form the basis of reagents for binding assays are labile molecules. When they are reacted to introduce the residue of a ligand, they tend to become inactive and lose their properties of immunoreaction. According to this invention, however, the reagents and the conjugate for the binding assay and methods for using them results in retained activity of the binding agent. That is, no loss

of immunoreactivity in the case of antigens or antibodies.

In accordance with this invention, a multi-labelled fluorescence system is provided which achieves amplification without quenching. Such amplification from the system provides improved sensitivity without compromising advantages of this type of assay design or simplicity in performance. Avidin or streptavidin is linked to a carrier particle, where the particle is provided with more than fifteen amino groups. The presence of the amino groups provide for the multiple labelling of the particle. Contrary to observation with conventional probes, multiple labelling with BCPDA of a carrier particle to linked avidin or streptavidin results in conjugates free from quenching effects.

For ease in discussing this invention, reference will be made specifically to the use of BCPDA as the ligand (label) although it is appreciated that any of the other selected compounds of formula I are useful as labels. As particularly used in the area of fluorescence with multiple labelling using for example BCPDA, it is thought that this amplification in fluorescence is due to the exceptional Stokes shift observed with such complexes with excitation at 325 nm and a sharp emission band at 615 mn. There is therefore essentially no overlap between the excitation and emission spectra.

Figure 1 illustrates three of the four types of assay systems, one of which is among the types contemplated by this invention. Systems (a) and (b) demonstrate systems of the aforementioned type, whereas system (c) demonstrates one of the embodiments of the invention for enhanced labelling system. The fourth system is shown in Figure 5. The system of Figure 1(c) will be discussed herein in detail before advancing to a detailed discussion of the system of Figure 5.

In system (a), the antibody directed to the α-fetoprotein (AFP) molecule is directly labelled with the ligand of Formula I in combination with europium. The system (b) has the antibody biotinylated and bound to streptavidin (or avidin) molecules having the ligand of Formula 1 in combination with europium on the streptavidin molecule. System (c) in accordance with this invention has the streptavidin molecule SA chemically linked to the carrier protein thyroglobulin (TG) which is in turn labelled a multiplicity of times with the ligand of Formula 1. The significant benefit of system (c) over that of (a) and (b) is demonstrated with respect to the following examples where it has been discovered that the amplification and hence fluorescent signal derived from this system greatly exceeds that possible with systems (a) and (b), hence, binding assays can be conducted to determine the presence of as little as 10 attomoles of α-fetoprotein.

There are, of course, many systems to which the conjugate and labelling system of this invention can be compared. For example, antibodies can be directly labelled with BCPDA or antibodies covalently bound to labelled bovine serum albumin (BSA) and used to devise an immunoassay for cortisol. Approximately 10 BCPDA molecules can be incorporated directly on the antibody, whereas approximately 40 BCPDA molecules can be incorporated on BSA covalently bound to an antibody. As a further technique, streptavidin, which has approximately 20 amino groups, can be labelled directly with about 14 BCPDA molecules and used preferably with biotinylated antibodies as complementary reagents. The labelled streptavidin results in an amplification of approximately 140 fold assuming a load of 10 biotin molecules per antibody which results effectively in a total of approximately 140 BCPDA molecules per analyte.

Contrary to the above, the conjugate according to this invention, comprises avidin or streptavidin conjugated to a carrier particle which may be a synthetic material of sub-micron size such as latex which can be provided with at least fifteen amino groups on its surface. This provides for a considerably higher loading of BCPDA on the protein or particle. Other types of support particles can be coated with proteinaceous material or the carrier particle may be a protein molecule. Suitable protein molecules may be selected from the group consisting of thyroglobulin, bovine serum albumin, hemocyanin, myosin, apoferritin, a series of polylysine co-polymers having different ratios of lysine/amino acid, $\alpha_2$-macroglobulin, leucine aminopeptidase, heavy meromyosin and histones.

A consideration in selecting a suitable protein molecule or other type of synthetic particle is that the carrier particle is capable of being chemically linked to avidin or streptavidin to form the conjugate. Acceptable forms of latex particles which are useful with this invention, may be obtained from Seragen Diagnostics Inc, of Indianapolis, Indiana. By use of a suitable carrier particle, multiple labels are readily provided on the carrier surface in excess of fifteen and preferably in excess of one hundred per carrier particle. For example, with streptavidin conjugated to the carrier protein bovine thyroglobulin (TG) approximately one hundred and fifty BCPDA residues may be provided. The resulting streptavidin TG conjugate retains the ability to bind to biotinylated antibodies and can therefore be used for highly sensitive time resolved fluorescent immunoassays. The theoretical amplification factor for this type of molecule is approximately $10^4$ fold when considering the bonus effect from the multiple labelling. As experienced with this invention, this multiple labelled molecule does not result in any quenching effects. As will be discussed with reference to Figure 5, a streptavidin or avidin macromolecular complex can be formed in accordance with this invention, which has an even greater loading of BCPDA molecules.

According to another aspect of the invention, the fluorescent reagent system for use in an assay comprises the above conjugate having the avidin or streptavidin suitably linked by biotin to an assay component. The carrier particle is labelled with a plurality of moieties of ligand forming with a lanthanide metal ion, a fluorescent chelate which are attached to the plurality of amine groups to provide at least fifteen labels and preferably in excess of one hundred labels. Each of the fluorescent chelate moieties has stably retained thereon a selected lanthanide metal ion.

The fluorescent system, according to this invention, when excited to fluoresce, emits radiation with an

intensity amplified compared to intensity of radiation emitted by the plurality of fluorescent lanthanide metal ion chelate moieties in a solution. Hence, the fluorescent reagent is void of any quenching effects due to multiple labelling as experienced in the past. Desired lanthanide metal ions to be incorporated in the fluorescent chelates, are selected from the group consisting of europium, terbium, gadolinium, samarium and dysprosium.

The fluorescent reagent system of this invention may be used in a binding assay. The binder has a plurality of the conjugates chemically linked thereto by a corresponding plurality of linking components. As noted, the carrier particle is labelled with a plurality of moieties of a ligand forming with the lanthanide metal ion, a fluorescent chelate. Each of the linking components comprises a biotin molecule chemically linked to the binder and in turn non-covalently linked to the avidin or streptavidin of the conjugate. A range of binders are useful in a variety of assays, such as in the field of immunoassays. Suitable binders include antibodies, folate binding protein, intrinsic factor, thyroxine binding protein, steroid binding protein, antigens and haptens. For specific types of immunoassays, monoclonal antibodies are preferred although the system works equally well with polyclonal antibodies.

It is preferred to use the streptavidin molecule in combination with the biotin. Although it is appreciated that avidin is also very useful. Streptavidin, as used in the binding assay, can be activated with sulfosuccinimidyl 4-(N-maleimidomethyl) cyclohexane-1-carboxylate (Sulfo-SMCC). Such activation is prior to the covalent bonding of the streptavidin to one of the amine groups of the carrier particle. In turn, the carrier particle can be activated with N-succinimidyl S-acetylthioacetate (SATA) and labelled extensively with BCPDA prior to chemical reaction with the activated streptavidin. The individual steps of this conjugation reaction are illustrated as follows to demonstrate the chemistry involved.

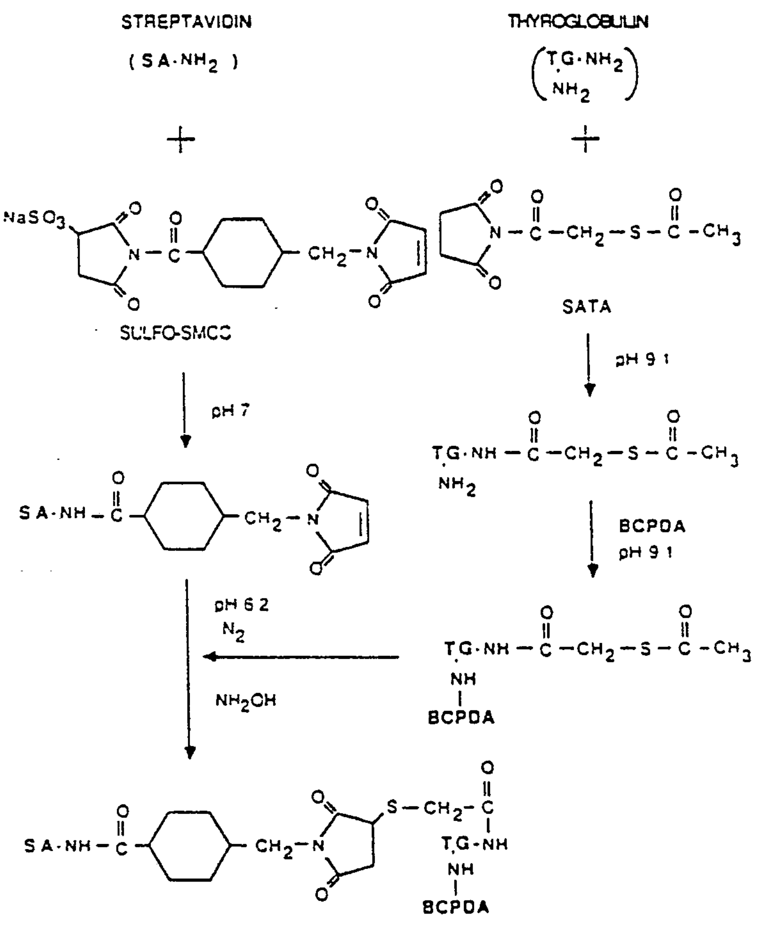


The initiation of the conjugation reaction occurs upon the addition of hydroxylamine which hydrolyzes the thioester and generates the thiol group which then reacts with the maleimide on the streptavidin forming the protein conjugate. According to this preferred embodiment, this reaction scheme is very useful in forming the conjugates using the sulfo-SMCC and the SATA for the following reason: The BCPDA labelling reaction is optimal at a pH of 9.1. Maleimides are most stable below pH 7. In the situation where thyroglobulin is used, it cannot be first activated by conversion of some of its amino groups into maleimide derivatives prior to extensive labelling at pH 9.1 because the maleimide groups would undergo hydrolysis. Thus, SATA is initially used to convert approximately five percent of thyroglobulin amino groups into thiol esters. The degree of thiolation can be assessed by the assay of Grassetti DR, Murray JF., Arch Biochem. Biophys. 1967;119:41-9. By using 2,2′-dithiodipyridine. The subsequent reaction with BCPDA results in the incorporation of 160 fluors

as determined by its absorbance at 325 nm.

The SATA derivative of the BCPDA labelled thyroglobulin is preferably used within twenty-four hours to ensure that the incorporated thiols remain protected. Hydrolysis of the acetyl derivative with time, could result in homopolymerization of thyroglobulin through disulfide bond formation.

The activation conditions for SA with 1 equivalent of sulfo-SMCC per amino group results in the incorporation of 3 to 4 maleimide groups per SA molecule, as determined by the 2,2′-dithiodipyridine method, Grassetti DR, Murray JF, Arch. Biochem. Biophys. 1967;119:41-9. The resulting derivative of SA is relatively stable in phosphate buffer at pH 6.2. However, immediate usage ensures the integrity of the maleimide groups.

Preferably the conjugate reaction is conducted in a reaction medium which is free of oxygen. The reaction then proceeds under reducing conditions in order to allow the free thiols to react with the maleimide groups and minimize the formation of disulfide bonds. The addition of mercaptoethanol after two hours of conjugation results in blocking any remaining maleimides on streptavidin as well as reducing any disulfide bonds that may have formed between thyroglobulin molecules. The addition of N-ethylmaleimide results in the blocking of all the free thiols in the reaction mixture.

It is appreciated that other systems may be used to activate the linking of the conjugate to streptavidin or avidin. For example, instead of using sulfo-SMCC, N-succinimidyl-3-(2-pydiryldithio)propionate (SPDP) may be used in its place. Alternatively, SA is activated with the homobifunctional reagent p-benzoquinone to which the BCPDA-TG conjugate is directly added. BCPDA-TG conjugate can be activated with excess $NaIO_4$ and SA added and the reaction quenched with glycerol. These alternatives are discussed with respect to the following Preparation 7.

With reference to Figure 5, an activation process is schematically shown which involves incubation under controlled conditions of a mixture containing BCPDA-labelled streptavidin-thyroglobulin conjugate, free BCPDA-labelled thyroglobulin and an amount of $Eu^{3+}$. After incubation, a new streptavidin-based macromolecular complex (SBMC) is formed which can be isolated and used for immunoassays in a manner exactly the same as the BCPDA-labelled streptavidin thyroglobulin conjugate. However, a significant improvement in detection limit can be achieved in comparison to BCPDA-labelled streptavidin thyroglobulin conjugate and at least a 35-fold improvement in detection limit compared to directly-labelled streptavidin of Figure 1(b). The new macromolecular complex is not fully understood, but it is thought that it consists of an $Eu^{3+}$ nucleus complexed with BCPDA present on thyroglobulin which is either unconjugated or covalently conjugated to streptavidin. By the following Examples, we have demonstrated that a highly sensitive immunoassay for prolactin with a detection limit of 68 $\alpha$moles/cuvette can be devised with the new macromolecule.

The macromolecule is synthesized by incubating the SA-TG-$(BCPDA)_{150}$ conjugate in the presence of excess TG- $(BCPDA)_{150}$ in a molar amount that was five times over that of SA. The isolated macromolecule (SBMC) contains the SA-TG-$(BCPDA)_{150}$ conjugate and also contains free (unconjugated) TG-$(BCPDA)_{150}$ but in a molar amount of approximately 2.3 $\pm$ 0.2 fold excess in comparison to the SA-TG-$(BCPDA)_{150}$ conjugate. The macromolecule is formed by adding to the isolated SA-TG-(BCPDA) conjugate less than the stoichiometric amount of $Eu^{3+}$ relative to moles of BCPDA present on the TG molecules, and incubating the resulting mixture at an elevated temperature in the range of 50° C for a period of approximately 3 hours to form the SBMC. The optimal amount of $Eu^{3+}$ has been found to be in the range of 70% to 85% saturation of the BCPDA molecules. This is discussed further with reference to the Examples.

The stability of the diluted macromolecular SBMC has a limited shelf life in the presence of europium $Eu^{3+}$. The loss of activity of the SBMC is presumably through a slow precipitation process. The stability, however, of the undiluted complex of the SA-TG-$(BCPDA)_{150}$ conjugate with $TG(BCPDA)_{150}$ in the absence of $Eu^{3+}$ is quite acceptable and has a shelf life for at least one year.

In the process of forming the SBMC, it has been found that the preferred pH for the solution in which the complex is formed is in the range of 5 to 7 with an optimal pH at 6. The rate of complexation in forming the SBMC is determined to some extent by time and temperature. The temperature of the solution during complexation should not exceed 60° C and should not be much below room temperature. The preferred temperature of complexation is in the range of 25° C to 50° C. The time of complexation during this preferred temperature range varies from 48 hours at 25° C to 3 hours at 50° C.

Based on the investigations reported in the following Examples, the formation of SBMC appears to be more than a simple binding process of europium to BCPDA-labelled thyroglobulin molecules. The SBMC has a fairly consistent molecular composition and is precipitated by the addition of super optimal amounts of $Eu^{3+}$. It is clear that the SBMC carries more BCPDA molecules per streptavidin or avidin molecules than the non-enhanced SA-TG-$(BCPDA)_{150}$; hence providing an even greater sensitive detection reagent than the conjugate. The molecular weight of the isolated SBMC complex appears to be in the range of 2.5 to 3.0 x $10^6$, such complex having little or no unassociated TG-$(BCPDA)_{150}$. It appears that the composition of the SBMC is 1:2.3:480 with respect to streptavidin covalently linked to BCPDA-labelled thyroglobulin and BCPDA-labelled thyroglobulin and $Eu^{3+}$. From the following experiments, it is suggested that the addition of the $Eu^{3+}$ to enhance the streptavidin conjugate, the unconjugated-(free) TG-$(BCPDA)_{150}$ molecules present in excess in the mixture aggregate and combine with the conjugate SA-TG-$(BCPDA)_{150}$ molecules to form the macromolecular complex. This complex is stable in solution for a limited period if it possesses the appropriate negative charge, but of course, precipitates if the $Eu^{3+}$ concentration exceeds the optimum limit.

The macromolecular complex, in accordance with an embodiment of the invention, provides a very useful

fluorophor. Its preparation is relatively simple and can be used in the same manner as all other embodiments of the invention without the need of any specialized procedure. The detection limit of the streptavidin based reagents insofar as the prolactin assay is concerned, as demonstrated in the following Examples, was found to be 3.14, 0.50 and 0.03 μg/l for the fluorophors of directly labelled streptavidin, SA-TG-$(BCPDA)_{150}$ conjugate and lastly, the SBMC. The SBMC with regard to the prolactin assay, measures approximately 68 (αmols) to provide a highly sensitive immunoassay using specific biotinylated antibodies as complementary reagents.

The binding assay system accorded to this invention can be adapted to detect a variety of biologically reactive matter. For example, the detection of a variety of haptens, antigen protein and antibody protein includes, cortisol, thyroxin, digoxin, biotin, α-fetoprotein (AFP), human chorionic gonadotropin (hCG), ferritin, thyrotropin (TSH) follitropin (FSH) lutropin (LH), thyroxine binding globulin (TBG) growth hormone or prolactin, antibodies or antigens specific to rubella virus or other infectious agents.

It is appreciated that the principles of the invention can be applied to other types of assays which may or may not be of the binding type. This technology is also applicable to a variety of receptor assays where a binding component of the receptor assay is labelled in accordance with this invention.

The following materials and reagents as identified are used in the following examples.

4,7-bis(chlorosulfophenyl)-1,10 phenanthroline 2,9-dicarboxylic acid (BCPDA) was synthesized as described previously, Evangelista RA, Pollak A, Allore B, Templeton EF, Morton RC, Diamandis EP. Clin. Biochem. 1988; sulfo-succinimidyl 4-(n-maleidomethyl) cyclohexane-1-carboxylate (sulfo-SMCC), was purchased from Pierce Chemical Co., Rockford IL 61105. N-succinimidyl S-acetylthioacetate (SATA) was obtained from Calbiochem Behring Diagnostics, La Jolla CA 92037. N-Ethylmaleimide was from Eastman Kodak Co., Rochester NY 14650. The concentration of proteins was carried out by using Centriprep™ and Centricon™ concentrators from Amicon Canada Ltd., Oakville Ontario L6H 2B9.

Fluoroimmunoassay microtitration plates, Microfluor™, white opaque 96-well plates were purchased from Dynatech Labs, Alexandria VA 22314. Affinity purified streptavidin and bovine thyroglobulin were obtained from Sigma Chemical Co., St. Louis, MO 63178. All other chemicals used were also from Sigma except where otherwise stated.

The monoclonal antibodies used for the α-fetoprotein and other immunoassays are available from Cyberfluor Inc.

Instrumentation

For measurement of solid-phase fluorescence we used the Cyberfluor 615™ Immunoanalyzer. The fluorescence of solutions was measured on the "Arcus" time-resolved fluorometer (LKB Wallac, Turku, Finland). Spectra were recorded on an HP Model 8450A diode array spectrophometer (Hewlett-Packard Canada, Mississauga, Ontario). Sephadex G-25 gel, and Sephacryl S-400 filtration columns, the pump, the optical unit and fraction collector were all from Pharmacia Canada, Dorval, Quebec H9P IH6. Ultrogel A34 was purchased from Fisher Scientific, Toronto, Ontario. The Speed Vac Concentrator was obtained from Emerston Instruments, Scarborough, Ontario M1R 1W4.

The following preparations and examples demonstrate preferred aspects of the invention.

Preparation 1

Derivatization of Thyroglobulin with SATA and Labelling With BCPDA

Fifty mg of bovine thyroglobulin [TG;M.W. 670,000 with 150 amino groups/molecule] were dissolved in 12 ml of a 0.1 M carbonate buffer of pH 9.1. TG was then initially derivatized with 1.6 mg of N-succinimidyl S-acetylthioacetate (SATA) dissolved in 80 μL of dimethylformamide; the SATA solution ( ~0.5 equivalent per amino group) was added dropwise in four portions to the stirring TG solution. The reaction was left to proceed for 1 h at room temperature. The protein was then extensively labelled with BCPDA by adding 40 mg of BCPDA dissolved in 500 μL of anhydrous ethanol (in five portions, dropwise) under continuous stirring. The mixture was stirred for 1 h at room temperature. The SATA derivatized, BCPDA labelled TG was isolated from unreacted SATA and BCPDA by extensive dialysis (M.W. cutoff 12-14,000) against a 0.1 M phosphate buffer of pH 6.2. The final product was then concentrated to approximately 0.3 mL using the Amicon concentrator.

Preparation 2

Streptavidin Activation With Sulfo-SMCC

One mg of streptavidin [SA;M.W. 60,000 with 20 amino groups/molecule] was dissolved in 200 μL of a 0.1 M phosphate buffer of pH 7.0. To this solution was added a trace amount of $^{125}$I-labelled streptavidin ( ~50,000 cpm/mg, obtained from Amersham Corporation, Oakville, Ontario L6H 2R3) to serve as a tracer for the accurate quantitation of the conjugation reaction. Streptavidin was then activated by the addition of 0.22 mg of sulfo-SMCC ( ~1.5 equivalents per amino group) dissolved in 50 μL of a 0.1 M phosphate buffer of pH 7.0 to the continuously stirred streptavidin solution. The reaction was allowed to proceed for 1 h at room temperature. The derivatized protein was isolated on a pre-equilibrated Sephadex™ G-25 column (1 x 15 cm) that was eluted with a 0.1 M phosphate buffer pH 6.2. The protein peak eluting near the void volume of the column was pooled and concentrated to approximately 0.1 mL with the Amicon Centricon Concentrator.

Preparation 3

Conjugation of Activated Streptavidin to Labelled Thyroglobulin

The derivatized TG and streptavidin solutions were mixed in a glass pyrex screw-cap test tube. The approximate molar ratio of TG:SA was about 5. The volume was then reduced down to 250-300 µL by repeating the following cycle three times. The solution was purged with $N_2$ gas for 30 s followed by centrifugation for 3 min in the Speed-Vac concentrator under reduced pressure. Vacuum was released under nitrogen. The conjugation was then initiated by the addition of 50 µL of a 0.5 M $NH_4OH$ solution of pH 7.0 that had been purged with $N_2$ for at least 15 minutes. The reaction mixture is the incubated for 2 h at 37° C under $N_2$. After incubation, 10 µL of a 0.12 M B-mercaptoethanol solution were added and the mixture incubated for 15 min at room temperature. Subsequently, 10 µL of a 0.24 M N-ethylmaleimide solution were added and the mixture incubated. for another 15 min. The streptavidin-thyroglobulin-BCPDA conjugate can then be isolated immediately or the next day after storage at 4° C, as described below. For purposes of subsequent discussion of the conjugate it will be identified as SA-TG-$(BCPDA)_n$ where n indicates multiple label of greater than 15.

Preparation 4

Conjugate Isolation

The reaction mixture is diluted with a 0.1 M bicarbonate solution of pH 8.0 to a final volume of 2 mL. An aliquot of the reaction mixture containing 200 µg of SA and 11 mg of BCPDA labelled TG was applied to an Ultrogel A34 column (3 x 42 cm) that was equilibrated and eluted with a 0.1 M $NaHCO_3$ solution, pH 8, at a flow rate of 1 mL/min. The optical density of the effluent was monitored at 280 nm. The volume of each fraction was 5 mL. The symbols I and II (Figure 2) denote the position at which BCPDA labelled TG and unconjugated SA, respectively, elute form this column under identical conditions. The 5 mL fractions were collected and counted on a gamma counter (from LKB Wallac). The fractions obtained near the void volume of the column were also assessed for their binding activity on microtiter wells coated with biotinylated antibody (solid-phase binding assay) and in a complete αfetoprotein assay. For these assays, an aliquot of the fractions was diluted to approximately 0.3 µg/mL in terms of streptavidin concentration using the radioactivity counts as the point of reference. The diluent was a 50 mM Tris buffer of pH 7.20 containing $Eu^{3+}$ (4 x $10^{-5}$ M), BSA (40 g/L) and sodium azide (0.5 g/L). The active fractions (in terms of binding to the biotinylated antibody) were pooled and diluted to 15 µg/mL in terms of streptavidin concentration using radioactivity as reference, in a 50 mM acetate buffer of pH 6.0, or 75 mM 2-(N-morpholino)ethane sulfonic acid, pH 6.0. This solution was stored at 4° C and diluted 50-fold in the europium containing diluent when it was used for an assay. From the procedure described above approximately 50 mL of concentrate can be obtained (sufficient for about 50,000 assays).

Preparation 5

Methodology for enhancing Fluorescence of Streptavidin Conjugate

Streptavidin-thyroglobulin conjugate of Preparation 4, as isolated from the Ultrogel A34 column, is diluted to 15 µg/ml in a solution buffered to pH 6 containing either 75 mM of 2-(N-morpholino)ethanesulfonic acid (MES) or 3-(N-morpholino-propanesulfonic acid (MOPS). A solution containing 1 mM $EuCl_3$ in 10 mM HCl was adjusted to pH 5.0 with NaOH and then added dropwise to the conjugate solution which was continually stirred during the addition. The amount of $Eu^{3+}$ added to the solution and its final concentration is dependent on the concentration of conjugated BCPDA that is present in the conjugated streptavidin solution. For the above conditions, the required $Eu^{3+}$ concentration is approximately 80% ± 5% of the BCPDA concentration as determined by the absorbance at 325 nm. After the $Eu^{3+}$ addition, the solution is incubated at 37° C for 16 h. The resulting mixture is then passed through a 0.45 µm filter thus affording a stock solution of streptavidin thyroglobulin conjugate loaded with BCPDA and $Eu^{3+}$.

Preparation 6

Alternate Methodology for Enhancing Fluorescence of Streptavidin Conjugate

This method utilizes the same solutions and procedure as outlined for Preparation 5, except that the incubation time after the addition of $Eu^{3+}$ is 3 hours and the incubation temperature is 50° C. The resulting solution is cooled at 4° C for at least 1 hour prior to filtering through a 0.45 µm filter, thus affording a stock solution of streptavidin thyroglobulin conjugate loaded with BCPDA and $Eu^{3+}$. Higher incubation temperatures were not advantageous due to the inactivation of the streptavidin and the precipitation of the conjugate. The resultant enhanced fluorescence conjugate is the streptavidin-based macromolecular complex (SBMC).

Preparation 7

The following Table summarizes the utility of various proteins which are readily labelled with BCPDA.

TABLE 1

| Protein | # of Amino Groups | Molecular Weight (x $10^{-3}$) | Molecular Ratio BCPDA:Protein[1] | | Relative Activity of BCPDA-Protein-SA Conjugate[2] |
|---|---|---|---|---|---|
| | | | Absorb | Fluor | |
| BSA | 59 | 66 | 40 | 260 | 0.5 |
| TG | 150 | 660 | 175 | 1000 | 5 |
| Cat | 169 | 250 | 122 | 261 | not tested |
| Fer | 194 | 480 | 190 | 208 | 0.5 |
| HC | >300 | >600 | 340 | 900 | 4 |

1 - The proteins were maximally labelled with 5 equivalents of BCPDA per free amino group on the protein in 0.1 M $Na_2CO_3$, pH 9.1. The modified proteins were isolated on a Sephadex ™ G-25 column and the extent of labelling assessed by the absorbance of the label at 325 nm and by its fluorescence in the presence of excess $Eu^{3+}$.

2 - These carrier molecules were individually conjugated to sulfo-SMCC activated SA and isolated on a Ultrogel A34 column. The relative activity of the conjugates were assessed through a binding assay of hCG, as compared with directly BCPDA labelled SA.

The above proteins were studied as possible BCPDA carrier molecules: bovine serum albumin; BSA, bovine thyroglobulin; TG, bovine liver catalase; Cat, horse spleen apoferritin; Fer, and keyhole limpet hemocyanin; HC.

The literature values for the number of amino groups and the molecular weight of the proteins were obtained from Peters (1975) for BSA, from Mercken et al (1985) for TG, from Tristram and Smith (1963) for Cat and Fer, and from Nakashima et al (1986) for HC.

All of the above proteins provided a degree of amplification in extent of fluorescence compared to number of labels present under the "Absorb" column which is BCPDA label without $Eu^{3+}$ present. The relative activity of these proteins as fluorescent labels all indicate an increase in radiation except for the catalase which was not tested. The thyroglobulin and hemocyanin proved to be particularly active.

Preparation 8

Labelled thyroglobulin may be conjugated to streptavidin in a variety of ways involving different types of activating chemicals. The following Table 2 identifies examples of various activating chemicals which may be used.

TABLE 2

Methods of Conjugation of BCPDA-TG to SA

| Method | Molar Ratio TG:SA in Reaction | Conjugation (% of SA) | Relative Activity[1] |
|---|---|---|---|
| sulfo-SMCC/ SATA | 2 | 75 | 1.0 |
| | 5 | 90 | 1.0 |
| SPDP/ SATA | 4 | 90 | 0.8 |
| p-Benzoquinone | 2 | 44 | 1.2 |
| | 5 | 50 | 2.1 |
| $NaIO_4$/ glycerol | 2 | 13 | 1.7 |
| | 5 | 17 | 1.6 |

1 - The relative activity of the conjugate was assessed on a binding assay for hGC

The formation of the BCPDA-TG conjugate was achieved by employing one of the following methods: 1. Activation of the SA by sulfo-SMCC and the incorporation of a thiol ester onto TG prior to extensive labelling

with BCPDA, as outlined in Preparations 1 and 2. 2. Similar methodology as 1, except that SA is activated with N-succinimidyl-3-(2-pyridyldithio)propionate (SPDP). 3. Activation of SA with the homobifunctional reagent p-benzoquinone to which BCPDA-TG is then added. 4. BCPDA-TG is activated with excess $NaIO_4$, SA is then added and the reaction is quenched with glycerol. The conjugates were isolated by gel filtration and the extent of conjugation was determined by utilizing $^{125}$I-SA as a tracer.

Example 1

Methods

The α-fetoprotein monoclonal detection antibody was labelled with BCPDA, isolated, characterized, and diluted to 5 mg/L. Coating of the microtitration strips with the α-fetoprotein monoclonal antibody, and the biotinylation procedure of the second antibody were in accordance with standard procedure.

Streptavidin was labelled with BCPDA. By this method 14 ± 1 BCPDA molecules are conjugated to one streptavidin molecule. The labelled streptavidin working solution contained 3 mg/L streptavidin and 4 x $10^{-5}$ mol/L $Eu^{3+}$ in a 50 mM Tris buffer at pH 7.20 containing 9 g NaCl, 40 g BSA and 0.5 g sodium azide per liter. In all cases, the extent of BCPDA labelling was assessed by absorbance measurements at 325 nm, where only BCPDA absorbs (molar extinction coefficient of 1.5 x $10^4$ $M^{-1}$ $cm^{-1}$). Characterization of the labelled protein by fluorescence was performed as follows: BCPDA was hydrolyzed by incubating a weighed amount in 0.1 M carbonate buffer of pH 9.50, at 40° C for 2 h. This stock solution ($10^{-4}$ M) was diluted appropriately to cover the range between $10^{-7}$ and 3 x $10^{-12}$ M. The dilution buffer was 10 mM Tris buffer of pH 7.80, containing $10^{-6}$ M $Eu^{3+}$. A standard curve was then constructed by plotting the fluorescence reading of each solution (Arcus fluorometer, 200 µL/well) versus the concentration of the standard BCPDA solution. BCPDA labelled protein was then diluted in the same buffer and the fluorescence measured as above. The BCPDA concentration could then be determined from the calibration curve.

Example 2

Solid Phase Binding Assay

White opaque microtiter wells were coated with 0,25,50,100,150 and 200 ng of biotinylated goat anti-human IgM antibody (from Tago Inc., Burlinghame, CA 94010) by adding 100 µL/well in a 0.1 M bicarbonate solution and incubating overnight at 4° C. The wells were then washed with a solution containing 9 g NaCl and 0.5 g Tween 20 per liter and blocked for 4 h at room temperature by adding 100 µL/well of a 0.1 bicarbonate solution containing 10 g/L bovine serum albumin (BSA). The wells were then rewashed and shaken dry. BCPDA labelled SA or SA-TG-$(BCPDA)_n$ conjugate was diluted in 50 mM Tris, pH 7.2 containing 4% BSA, 0.9% NaCl 0.05% $NaN_3$ and 40 µM $Eu^{3+}$, to yield different concentrations (in terms of streptavidin) as follows and which are referenced in Figure 3: 0.33 µg/mL (triangle), 0.5 µg/mL (diamonds), 1.0 µg/mL (squares) or 3.0 µg/mL (circles). One hundred µL of each solution of BCPDA-SA (open symbols) or SA-TG-$(BCPDA)_n$ (closed symbols) was applied in duplicate to each of the microtitre wells containing varying amounts of the biotinylated antibody and incubated for one hour at 37° C. The plates were then washed dried and the fluorescence counts measured with the Cyberfluor 615™ fluorometer. The fluorescence counts were plotted against the amount of biotinylated antibody immobilized on the well.

Example 3

α-Fetoprotein Assay

a) With directly labelled antibody 20 µL of α-fetoprotein standards were pipetted into the AFP antibody coated microtitrations wells. One hundred µL of the directly labelled antibody solutions (5 µg/mL of antibody, containing $10^{-5}$ M $Eu^{3+}$) were added and incubated for 90 min. at 37° C. The wells were then washed three times with the wash solution, dried for 3 min. with cool air and the fluorescence was measured on the Cyberfluor Model 615™ Immunoanalyzer.

b) With biotinylated antibody. The procedure is as above but utilizes the biotinylated second antibody instead of the directly labelled antibody. After washing, 100 µL of either directly labelled streptavidin solution (3 µg/mL), containing $Eu^{3+}$ (4 x $10^{-5}$ M), or the streptavidin thyroglobulin conjugate solution containing $Eu^{3+}$ (4 x $10^{-5}$ M, diluted as described above were added to the wells and incubated for 30 min at 37°C. After an additional three washes, the wells were dried and measured on the CyberFluor Model 615™ Immunoanalyzer, as described above. Calibration curves for the α-fetoprotein assay are shown in Figure 4 with directly labelled antibody (A) or biotinylated antibody and directly labelled streptavidin (B) or streptavidin conjugated to labelled thyroglobulin (C).

Example 4

The procedure of Example 3 is followed to determine detection sensitivities for streptavidin labelled with BCPDA, streptavidin-thyroglobulin conjugate labelled with BCPDA of Preparation 4 and the $Eu^{3+}$ carrying conjugate of Preparation 5. The reactive matter which is detected is thyrotropin (TSH), human chorionic gonadotropin (hCG) and α-fetoprotein (AFP). The parameters of the method are itemized in the following Table 3.

TABLE 3

Assay Procedures

| | TSH | hCG | AFP |
|---|---|---|---|
| Sample volume | 200 µl | 50 µl | 20µl |
| Biotinylated antibody volume | 50 µl | 50 µl | 100µl |
| Incubation time wash | 4 hours | 1 hour | 1.5 hours |
| Labelled streptavidin volume or streptavidinthyroglobulin of Preparation 4 or 5 | 100 µl | 100 µl | 100 µl |
| Incubation time wash | 30 min. | 45 min. | 45 min. |

The sensitivities of these carrier-labelled components as determined by the above procedures are itemized in the following Table 4.

TABLE 4

Achievable Sensitivities with Reagents Labelled with BCPDA

| Reagents[1] | Detection Limit TSH, mU/L | hCG, U/L | APF, ng/mL |
|---|---|---|---|
| Streptavidin $(BCPDA)_{15}$ | 1 | 5 | 0.2 |
| Streptavidin-(TG)-$(BCPDA)_{150}$ | 0.15 | 1 | 0.05 |
| Enhanced Streptavidin-(TG-) $(BCPDA)_{150}$ | 0.03 | 0.2 | 0.01 |

1. Biotinylated monoclonal antibodies are used as complementary reagents.

TG = bovine thyroglobulin

The isolation of the SA-TG-$(BCPDA)_{150}$ conjugate employs the molecular sieving properties of the Ultrogel A34 column which is capable of fractioning proteins having molecular weights for 1 x $10^4$ to 7.5 x $10^5$ (Figure 2). This column affords a large separation between the conjugated and the unconjugated SA; SA-TG-$(BCPDA)_{150}$ would have a molecular weight that exceeds 8 x $10^5$ as compared to 6 x $10^4$ for SA. This separation was monitored by the radioactivity that was associated only with SA. The presence of $^{125}I$ radioactivity near the void volume of this column proves that SA has been conjugated to the TG-$(BCPDA)_{150}$. SA contains no free thiol group and, therefore, the activated SA could not form a homopolymer of high molecular weight.

This column does not separate the unconjugated TG-$(BCPDA)_{150}$ from that covalently bound to SA, as indicated in Figure 2. However, elaborate methods of purification of SA-TG-$(BCPDA)_{150}$ from TG-$(BCPDA)_{150}$, such as with an immunobiotin column, were not necessary. We found that the excess TG-$(BCPDA)_{150}$ does not contribute significantly to the overall background fluorescence observed in an α-fetoprotein assay which was used as the working model.

Figure 3 illustrates the improved sensitivity obtained with the SA-TG-$(BCPDA)_{150}$ conjugate in comparison

to directly labelled SA in studies of binding to microtiter wells that are coated with biotinylated antibodies. The fluorescence reading observed with the conjugate are at least five times higher than those obtained with BCPDA labelled streptavidin at the same concentration.

During the developmental stage of our investigation, we tried a number of other chemical conjugation procedures. We have used the periodate conjugation method, as reported in Tijssen, P., Practice and Theory of Enzyme Immunoassays, Amsterdam: Elsevier, 1985, after activation of BCPDA labelled TG with $NaIO_4$ to generate aldehyde groups on the sugar moieties of TG. The aldehyde groups were then reacted with the amino groups of SA and the Schiff's base stabilized by reduction with sodium borohydride or addition of glycerol. We have also used the well-known homobifunctional reagent benzoquinone and the newer reagent N-succinimidyl-3-(2-pyridyldithio) propionate (SPDP). In all cases active conjugates were produced. However, the yields were lower than those obtained with sulfo-SMCC/SATA procedure. (Table 2)

We have investigated a number of proteins other than TG to serve as carriers of BCPDA. As previously noted, these proteins include BSA, hemocyanin, myosin, ferritin, catalase and a series of polylysine copolymers having different ratios of lysine/amino acid and different molecular weights. These carrier proteins were selected because they have a large number of amino groups so as to carry as many BCPDA molecules as possible. We found that of the proteins tested, TG conjugates gave the best signals in the solid-phase assay and the AFP sandwich assay (see below) as compared to conjugates with BSA, hemocyanin and ferritin.

The stability of the SA-TG-$(BCPDA)_{150}$ conjugate was assessed at 4° C and 37° C. We found the stability to be best when the conjugate is stored as a relatively concentrated solution (15 µg/mL in terms of streptavidin) in a 0.05 M acetate buffer or 75 mM MES at pH 6.0. This solution is usually diluted 50 fold in a 50 mM Tris buffer of pH 7.20, containing 40 g BSA per liter and $Eu^{3+}$ ($4x10^{-5}$ M) just before use in an assay. Stored as a concentrate, the conjugate was found to be stable for at least 12 months at 4° C. Accelerated stability studies showed that the conjugate was stable for at least 2 weeks at 37° C. According to the literature, this finding suggests that the SA-TG-$(BCPDA)_{150}$ conjugate is stable for at least 2 years at 4° C . After dilution, the reagent is stable for one day at room temperature making it convenient for routine use.

We have developed an immunoassay procedure for α-fetoprotein in serum using two monoclonal antibodies. One antibody is immobilized in white opaque microtitration wells to form the solid-phase. The second antibody (detection antibody) is either directly labelled with BCPDA for a cortisol monoclonal antibody or biotinylated. The molar ratio of BCPDA to the antibody as determined by absorbance measurements at 325 nm was 10.

We used three different methods of performing the AFP assay, as illustrated in Figure 4, in order to demonstrate the amplification of each system: (a) directly labelled antibody (b) biotinylated detection antibody in conjunction with directly labelled streptavidin (c) biotinylated detection antibody in conjunction with streptavidin conjugated to labelled thyroglobulin, as described above. The calibration curves are shown in Figure 4. We calculated the detection limit in each case [defined as the concentration of AFP which can be distinguished from zero with 95% confidence] and found to be 5, 1 and 0.2 ng/mL, respectively. The improvement in signal of system (b) over system (a) is approximately 15-fold. The detection limit improves only approximately 5-fold because the background signal (obtained with the zero standard) due to non-specific absorption to the wells of the reagents was higher in system (b) than it was in system (a). The improvement in signal of system (c) over system (b) is about 5-fold and so is the improvement in the detection limit. Background signals in systems (b) and (c) were similar. Furthermore, the enhanced SA-TG-$(BCPDA)_{150}$ exhibits an additional 5 fold improvement in signal over the non-enhanced conjugate of system (c) as demonstrated in Table 4.

The streptavidin-based macromolecular complex (SBMC) having enhanced fluorescence is used in a prolactin assay to determine its utility.

Example 5

Fifty µL of prolactin standards were pipetted into the prolactin antibody coated microtitration wells and were incubated at room temperature with shaking for 1 hour. One hundred and fifty µL of the biotinylated second antibody were then added and incubated for an additional 1 hour at room temperature with shaking. The wells were then washed three times with the wash solution. After washing, 100 µL of either directly labelled streptavidin solution or the streptavidin thyroglobulin conjugate solution, (either enhanced or non-enhanced, see below) diluted as described above, were added to the wells and incubated for 30 minutes at 37° C. After an additional three washes, the wells were dried and measured on the Cyberfluor Model 615® Immunoanalyzer.

Example 6

The $Eu^{3+}$ enhanced SBMC or the non-enhanced streptavidin thyroglobulin conjugate SA-TG-$(BCPDA)_{150}$ was diluted to 0.3 mg/L with respect to streptavidin in a solution which contained 4 x $10^{-5}$ mol/L $Eu^{3+}$ in a 50 mM Tris buffer at pH 7.20 containing 9 g NaCl, 40g BSA and 0.5 g sodium azide per liter. One hundred mL of this working solution was then added to each well of the prolactin during the second incubation step, as described in Example 5.

Example 7

The SBMC was isolated from any free BCPDA-labelled thyroglobulin by gel filtration chromatography on a Sephacryl S-400® column (2.6 x 35 cm). These chromatograms were obtained when approximately 0.8 nmoles

of SA-TG-(BCPDA)$_{150}$ and 3.3 nmoles of TG-(BCPDA)$_{150}$ were applied to the column that was equilibrated and eluted with 60 mM MES, pH 6, at a flow rate of 0.4 mL/min before (- - -) and after (—) a 3 hour incubation with $Eu^{3+}$ (76% of the BCPDA present) in 75 mM MES, pH 6 at 50° C. The 325 nm absorbance, the radioactivity ($^{125}$I-SA) and the relative fluorescence signal on the prolactin assay of the isolated SBMC was quantitated. The SBMC was then broken down into its constituents (streptavidin covalently linked to BCPDA-labelled thyroglobulin; BCPDA-labelled thyroglobulin; and $Eu^{3+}$) by the following procedure. The SBMC was incubated overnight at room temperature with EDTA in $Na_2CO_3$, pH 9.1, in a molar amount that was 30 times that of the BCPDA present. The resulting mixture was applied to a Sephadex G-25 column and eluted with 0.1 M $NaHCO_3$, pH 8 (Figure 10). The eluate was monitored at 280 nm and the amount of $Eu^{3+}$ in each fraction was assessed by diluting an aliquot of each fraction 100-fold in 100 mM acetate buffer, pH 3.5, then pipetting 10 µL into a clear microtitre strip to which 200 µL of an Enhancement Solution (provided commercially by LKB Wallac) was added and the fluorescence measured on the Arcus fluorometer. The $Eu^{3+}$ concentration was then quantitated based on a standard curve that was generated using an identical procedure. The protein peak eluting near the void volume of the column was pooled and the absorbance at 325 nm, the radioactivity ($^{125}$I-SA) and the relative fluorescence signal on the prolactin assay was determined.

Various conjugate solutions were assessed as a detection reagent in conjunction with biotinylated antibodies, on a complete prolactin assay of Example 5 where a 25 µg/L standard prolactin solution was used. The detection reagent involved the dilution of the conjugate solutions in a $Eu^{3+}$ containing buffer as described in Example 5. The results are summarized in Figure 6. Assessment of the SA-TG-(BCPDA)$_{150}$ conjugate after incubation in 75 mM MES, pH 6 at 50°C for 3 hours, with excess TG-(BCPDA)$_{150}$ and in the presence of $Eu^{3+}$. The amount of $Eu^{3+}$ added was expressed as a molar ratio to the BCPDA molecules covalently linked to TG. The product was assessed by its absorbance at 325 nm ( □ ) and its relative fluorescence signal (relative tracer activity) for a standard solution containing 25 mg/L prolactin in a time-resolved fluorescence immunoassay (■). For more details, definitions and discussions, see the text. It can be seen that, by increasing the amount of $Eu^{3+}$ up to a certain point (65% of the required equivalent concentration for 1:1 stoichiometry in relation to BCPDA concentration) the absorbance of BCPDA remains relatively constant. After this point, increasing amounts of $Eu^{3+}$ cause a decrease in BCPDA absorbance because of SA-TG-(BCPDA)$_{150}$ and free TG-(BCPDA)$_{150}$ precipitation (which is also identified visibly). In Figure 6, the relative activity of the $Eu^{3+}$ enhanced conjugate is shown. The relative activity is expressed as follows: an activity of 1.00 is arbitrarily defined for the fluorescence reading observed with a 25 µg/L prolactin standard in a complete prolactin assay, when the conjugate SA-TG-(BCPDA)$_{150}$ is used without activation with $Eu^{3+}$ at 50° C. That is, the conjugate is used as described previously. After each $Eu^{3+}$ addition, the relative activity was calculated by using the ratio of observed fluorescence with $Eu^{3+}$ enhancement over the fluorescence observed without $Eu^{3+}$ enhancement.

It can be seen that a maximum of 7.2-fold amplification in signal can be achieved by incubating with an optimum amount of $Eu^{3+}$. However, $Eu^{3+}$ addition that is over the optimal, results in conjugate precipitation and the subsequent loss of activity (Figure 6). The optimum $Eu^{3+}$ concentration in the mixture which is suitable to give maximum amplification lies in a very narrow range (Figure 6); therefore, it is preferable to empirically titrate aliquots of the initial conjugate with $Eu^{3+}$ in the range of 70% to 85% saturation of BCPDA and test the aliquots in a complete assay.

In Figure 7, complete prolactin calibration curves are presented in the range of 0.1 to 25 µg/L by using (a) streptavidin directly labelled with 14 BCPDA molecules (curve 1); (b) SA-TG-(BCPDA)$_{150}$ conjugate without $Eu^{3+}$ activation (curve 3) and (c) SBMC but with varying amounts of $Eu^{3+}$ (curve 2, 4-8). The calibration curves for the prolactin assay with directly BCPDA-labelled SA (1), SA-TG-(BCPDA)$_{150}$ conjugate (3) or the macromolecular complex that was generated upon incubation of SA-TG-(BCPDA)$_{150}$ with excess TG-(BCPDA)$_{150}$ and in the presence of $Eu^{3+}$. The amount of $Eu^{3+}$ added was 30% (4); 60% (6); 70% (7); 76% (8); 80% (5) or 85% (2) with respect to the BCPDA present (1:1 stoichiometry). It can be seen that maximum signal can be observed with the fraction of 76% BCPDA saturation by $Eu^{3+}$ (curve 8). The observed amplification over (a) and (b) is about 35-fold and 7-fold, respectively.

Fully activated fractions of the SBMC were stored at 4° C and tested frequently in complete immunoassays, to assess their stability. It was found that the stored concentrates (concentration of 15 mg/L in terms of streptavidin) are stable for at least 1 year. These concentrates are diluted 50-fold in a suitable buffer just before use in the assay; the buffer was 50 mmol/L Tris, pH 7.20, containing 9 g NaCl, 40 g BSA and 0.5 g sodium azide per liter to dilute the streptavidin preparations. This buffer must be supplemented with excess $Eu^{3+}$ ($4 \times 10^{-5}$ mol/L) when directly labelled streptavidin or untreated SA-TG-(BCPDA)$_{150}$ is used as a detection agent. Both the requirement of the maximally enhanced SMBC for $Eu^{3+}$ in the dilution buffer as well as the stability of the diluted SBMC with time in the presence or absence of $Eu^{3+}$ in the diluent (Figure 8) have been examined. Relative stability of the macromolecular complex after dilution into a 50 mM Tris buffer, pH 7.20, containing 40 g BSA, 9g NaCl and 0.5% sodium azide per liter, in the presence (■) or absence ( □ ) of 40 µM $EuCl^3$ is shown. This diluted complex was stored at 4° C and at the appropriate times was used in a time-resolved fluorescence prolactin immunoassay. The fluorescence signal obtained for a prolactin standard (5 µg/L) immediately after tracer dilution, was arbitrarily defined as 100%. As observed, the SBMC gives a similar signal and demonstrates much longer stability when diluted in the absence of $Eu^{3+}$. The diluted SBMC in the presence of excess $Eu^{3+}$, loses its activity with time, presumably through a slow precipitation process.

The $Eu^{3+}$ enhancement of the SA-TG-(BCPDA)$_{150}$ conjugate was investigated at various temperatures and

pH values. A pH of 6 was found to be the optimal value for conjugate enhancement though it would occur to a laser extent at pH 7 and 5 but not at a pH of 8. The temperature of enhancement primarily altered the rate of formation of the SBMC, i.e, completion of the enhancement would occur at about 48 hours, 16 hours and 3 hours for the temperatures of 24°C, 37°C and 50°C, respectively. Temperatures above 60°C generally decreased the activity of the conjugate.

Examination of the mechanism of the enhancement process has yielded the following observations: Conjugates of the form SA-[TG-$(BCPDA)_{150}]_3$ isolated by gel filtration on a Sephacryl S-400 column and thus not containing excess BCPDA-labelled thyroglobulin, cannot be enhanced by the addition of $Eu^{3+}$. Directly labelled streptavidin does not undergo any activation by titrating it with $Eu^{3+}$ even in the presence of excess BCPDA. Furthermore, no benefit was obtained when the amount of BCPDA-labelled thyroglobulin in the conjugation reaction was increased (i.e. increasing the molar ratio from 1:5 which we use routinely to 1:10 and 1:20).

These data suggest that (a) the formation of the new species after enhancement is not a simple binding process of $Eu^{3+}$ to BCPDA-labelled thyroglobulin molecules; (b) the new species has a precise molecular composition and precipitates by the addition of the superoptimal amount of $Eu^{3+}$; (c) the new species must carry more BCPDA molecules per streptavidin than the non-enhanced SA-TG-$(BCPDA)_{150}$, thus being a more sensitive detection reagent and (d) the observed amplification suggests the $Eu^{3+}$ may form with BCPDA complexes of the 1:2 type because complete utilization of unreacted BCPDA-labelled thyroglobulin could increase the signal by a maximum factor of 3.3. The experimentally-derived factor is around 7.

In Figure 9, a gel filtration chromatography experiment is presented. Elution behavior of the SA-TG-$(BCPDA)_{150}$ conjugate in the presence of excess TG-$(BCPDA)_{150}$ from a Sephacryl S-400 column (2.6 x 35 cm). The arrow denotes the position at which TG-$(BCPDA)_{150}$ elutes from this column under identical conditions. The dashed line represents the elution pattern of the mixture of the conjugation reaction between streptavidin and BCPDA-labelled thyroglobulin (initial molar ratio 1:5). There is a small peak eluting around 70 mL corresponding to a molecular weight of approximately 2.5-3.0 x $10^6$, followed by a continuous spectrum of absorbance up to 105 mL where there is a peak corresponding to a molecular weight of approximately 0.8 x $10^6$. The peak eluting around 70 mL is likely due to a conjugate of the form SA-[TG-$(BCPDA)_{150}]_3$ while between this volume and 105 mL another conjugate of the form SA-[TG-$(BCPDA)_{150}]_2$ may be eluting. The bulk of the material, however consists of SA-TG-$(BCPDA)_{150}$ and unconjugated TG-$(BCPDA)_{150}$ both eluting around 105 mL. By monitoring the radioactivity from $^{125}$I-streptavidin, incorporated as a marker in the conjugation mixture, we could examine that the approximate percentages of SA-[TG-$(BCPDA)_{150}]_3$: SA-[TG-$(BCPDA)_{150}]_2$: SA-TG-$(BCPDA)_{150}$ are 5:20:75. Isolated conjugates of the SA-[TG-$(BCPDA)_{150}]_2$ type do not become enhanced as $Eu^{3+}$ is added.

After the enhancement reaction was performed, the reaction mixture (initially corresponding to the dashed line of Figure 9) was rechromatographed on the same column generating the elution pattern represented by the solid-line. Little or no unreacted TG-$(BCPDA)_{150}$ could be seen, the major product now appearing as a macromolecular complex of approximately 2.5-3.0 x $10^6$ molecular weight.

Further investigation into the composition of the SBMC with respect to the ratio of streptavidin covalently linked to BCPDA-labelled thyroglobulin, BCPDA-labelled thyroglobulin and $Eu^{3+}$ was performed by incubating the isolate obtained from the Sephacryl S-400 column (the pooled protein peak having absorbance at 280 nm and eluting around 70 mL, Figure 9) with 30 times excess EDTA overnight at room temperature. The resulting solution was applied and eluted from a Sephadex G-25 column generating the chromatogram in Figure 10. This is the elution behavior of the SBMC in the presence of excess EDTA from a Sephadex G-25 column (1.6 x 36 cm). This chromatogram was obtained when approximately 0.25 moles of SBMC that had been incubated with EDTA overnight were applied to the column that was equilibrated and eluted with 0.1 M $NaHCO_3$, pH 8 at a flow rate of 1.0 mL/min. The eluate was monitored at 280 nm (—) and the amount of $Eu^{3+}$ in each fraction (approximately 2 mL volume) was quantitated on the Arcus time-resolved fluorometer (■).

The addition of EDTA should extract and complex the $Eu^{3+}$ present in the SBMC due to its superior affinity for $Eu^{3+}$ (Ka = $10^{-18}$) over that of BCPDA (Ka = $10^{-10}$). As observed in Figure 10, all of the protein eluted near the void column of the column and the recovery was 100% as determined by radioactivity ($^{125}$I-SA). Furthermore, the $Eu^{3+}$ was not associated with the protein but eluted in fractions closer to the bed volume of the column. The proportion of SA to BCPDA-TG in the pooled protein fractions was found to remain at the 1:3.3 ratio; however, its activity as a detection reagent for the prolactin assay is now correlated to the original non-enhanced SA-TG-$(BCPDA)_{150}$ conjugate and not to the SBMC. Therefore, the composition of the SBMC appears to be 1 : 2.3 : 480 with respect to streptavidin covalently linked to BCPDA-labelled thyroglobulin, BCPDA-labelled thyroglobulin and $Eu^{3+}$.

These results suggest the following mechanism of formation of the streptavidin based macromolecular complex (SBMC), although it is understood that the applicants do not wish to be bound by such theory. Upon the addition of $Eu^{3+}$, non-covalently conjugated, free TG-$(BCPDA)_{150}$ molecules present in excess in the mixture, aggregate and combine with SA-TG-$(BCPDA)_{150}$ molecules to form the macromolecular complex as shown in Figure 5. This complex appears to be stable if it possesses certain negative charge but precipitates if the $Eu^{3+}$ concentration exceeds a certain limit. The formation of this aggregate is relatively slow, but is complete after 3 hours incubation at 50°C.

In practical terms, the new streptavidin-based macromolecular complex is very useful. Its preparation is relatively simple. The actual reagent can be used in the same manner as the directly labelled streptavidin

preparations without the need to use any specialized procedures. Additionally, all the advantages of the time-resolved fluorescence immunoassay system that have been developed are still present with the use of this reagent (SBMC). The sensitivity of the methodologies developed using this reagent in comparison to the previous streptavidin-based reagents that have been described have been improved by a factor of at least 35-fold over labelled streptavidin, or 7-fold over SG-TG-$(BCPDA)_{150}$. The detection limit of the prolactin assay that was used in Example 5 as a model was found to be 3.14, 0.50 and 0.03 µg/L, for the detection system with directly labelled streptavidin, SA-TG-$(BCPDA)_{150}$ and the new macromolecular complex, respectively. Thus, with the enhanced reagent approximately 68 αmols of prolactin can be measured. The same reagent has been tested for the measurement of other analytes in biological fluids using biotinylated antibodies as complementary reagents. A similar beneficial effect in terms of detection limits was observed in all cases. The same reagent may be used in other area of research like immunohistochemistry and DNA probing.

Although preferred embodiments of the invention have described herein in detail, it will be understood by those skilled in the art that variations may be made thereto without departing form the spirit of the invention or the scope of the appended claims.

## Claims

1. A conjugate for use in a labelling system characterized by avidin or streptavidin linked to a carrier particle having more than 15 amino groups on its surface which are individually capable of being labelled with an operable label, said carrier particle being capable of being linked to avidin or streptavidin to form said conjugate.

2. A conjugate of claim 1 characterized by said particle being a synthetic sub micron size particle having said at least fifteen amine groups on the particle surface.

3. A conjugate of claim 1 characterized by said particle is a protein molecule having said amine groups on its surface.

4. A conjugate of any of the preceding claims, characterized by said carrier particle being linked to avidin.

5. A conjugate of any of the preceding claims, characterized by said carrier particle being linked to streptavidin.

6. A fluorescent reagent system for use in an assay characterized by a conjugate of claim 1 having said avidin or streptavidin suitably linked by biotin to an assay component, said carrier particle being labelled with a plurality of moieties of a ligand attached to said plurality of amine groups to provide at least 15 labels, each of said labels in the presence of a selected lanthanide metal ion forming a plurality of fluorescent chelates, said fluorescent reagent system when excited to fluoresce, emits fluorescent radiation with an intensity amplified compared to intensity of fluorescent radiation emitted by said plurality of said fluorescent lanthanide metal ion chelate moieties in a solution whereby said fluorescent reagent is void of any quenching effect due to the multiple labelling in a fluorescent reagent system.

7. A fluorescent reagent system of claim 6 characterized by said ligand is a derivative of 4,7-diphenyl-1,10-phenanthroline-2,9-dicarboxylic acid.

8. A fluorescent reagent system of claim 6 or 7 characterized by said lanthanide metal ion being selected from the group consisting of europium, terbium, gadolinium, samarium, and dysprosium.

9. A conjugate of any of claims 1 to 5 or a fluorescent reagent system of any of claims 6 to 8 characterized by said carrier particle being a protein selected form the group consisting of thyroglobulin, bovine serum albumin, hemocyanin, myosin, apoferritin, catalase, a series of polylysine copolymers having different ratios of lysine/amino acid, $\alpha_2$-macroglobulin, leucine-aminopeptidase, heavy meromyosin and histones.

10. A fluorescent reagent system for use in an assay characterized by a macromolecular complex of streptavidin or avidin complexed with a plurality of carrier particles each having more than 15 amino groups on its surface which are individually capable of being labelled with a fluorescent chelate of a ligand moiety with a lanthanide metal ion, the stoichiometric ratio of lanthanide metal ions to the amino groups on said plurality of particles being less than 1, said fluorescent reagent system when excited to fluoresce, emits fluorescent radiation with an intensity amplified compared to intensity of fluorescent radiation emitted by said plurality of said fluorescent lanthanide metal ion chelate moieties in a solution.

11. A fluorescent reagent system of claim 10, characterized by said lanthanide metal being europium.

12. A fluorescent reagent system of claim 10, or 11, characterized by said carrier particle being thyroglobulin.

13. A fluorescent reagent system of claim 10, 11 or 12, characteirzed by said ligand being a derivative of 4,7-diphenyl-1,10-phenathroline-2,9-dicarboxylic acid.

14. A fluorescent reagent system of claim 12, chracterized by said stoichiometric ratio of europium ions to said amino groups with said ligand moieties linked thereto being in the range of 0.75 to 0.85.

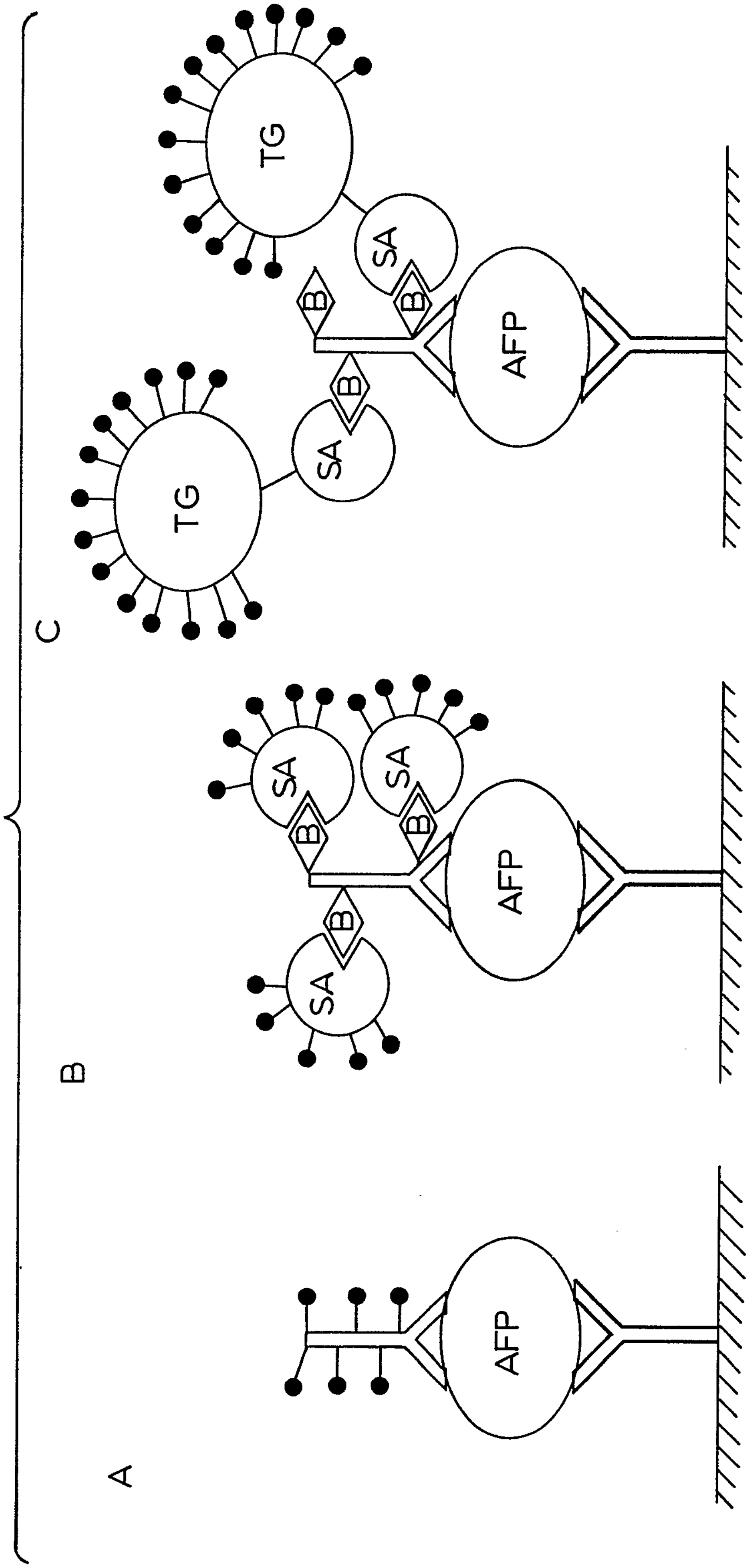


FIG. 1

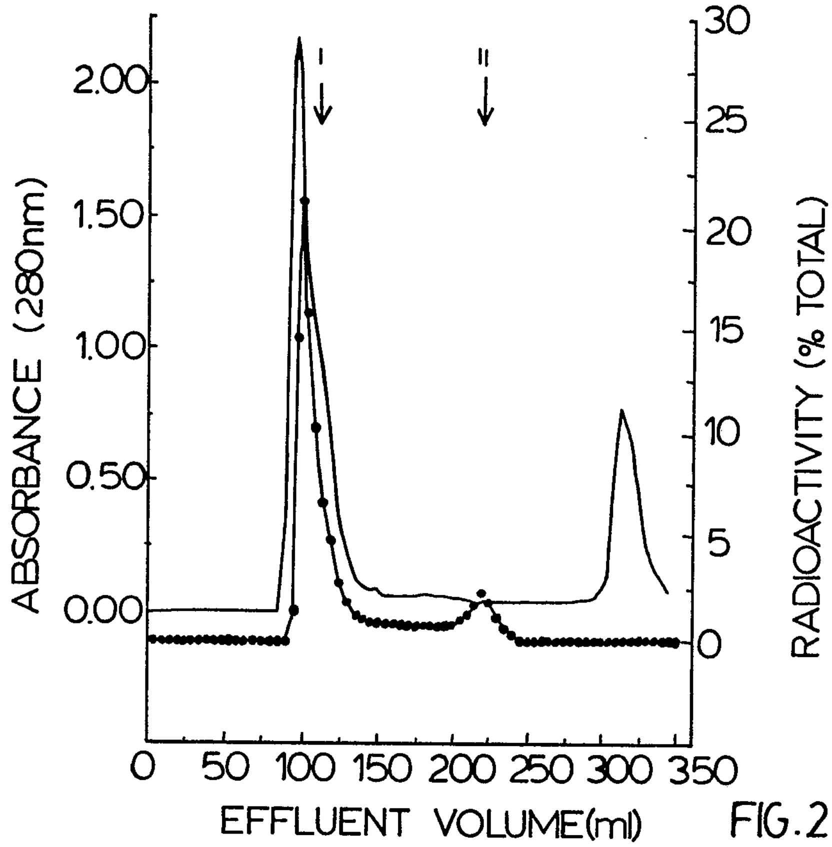

2.00
1.50
1.00
0.50
0.00
ABSORBANCE (280nm)
30
25
20
15
10
5
0
RADIOACTIVITY (% TOTAL)
I
II
0
50
100
150
200
250
300
350
EFFLUENT VOLUME(ml)


FIG.2

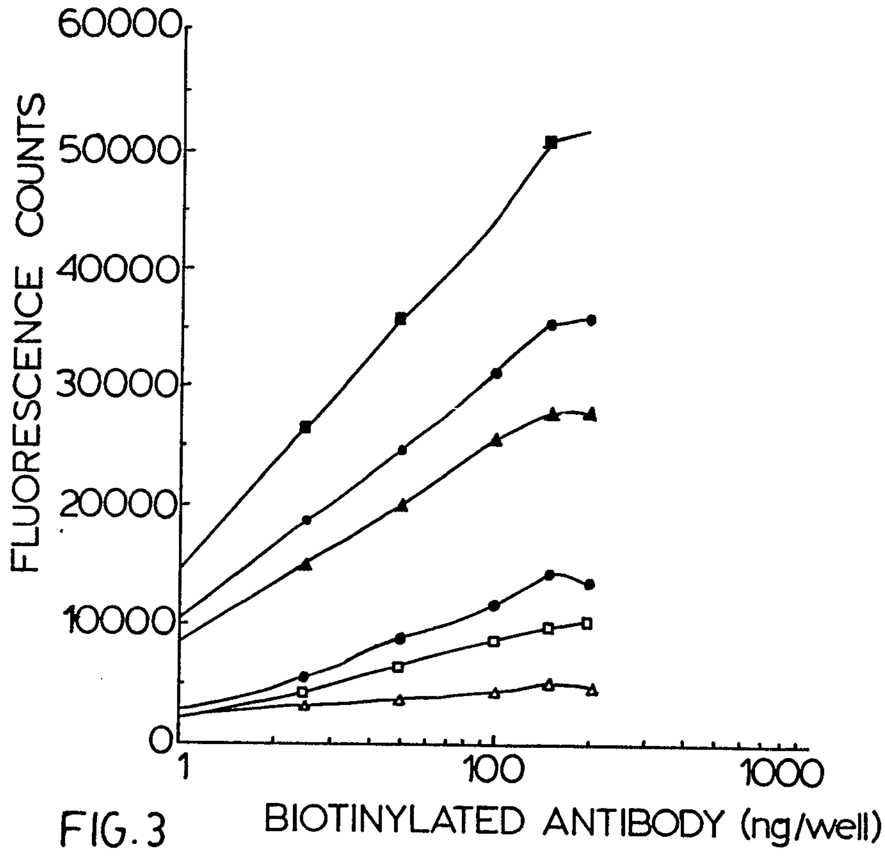

60000
50000
40000
30000
20000
10000
0
FLUORESCENCE COUNTS
1
100
1000
BIOTINYLATED ANTIBODY (ng/well)


FIG.3

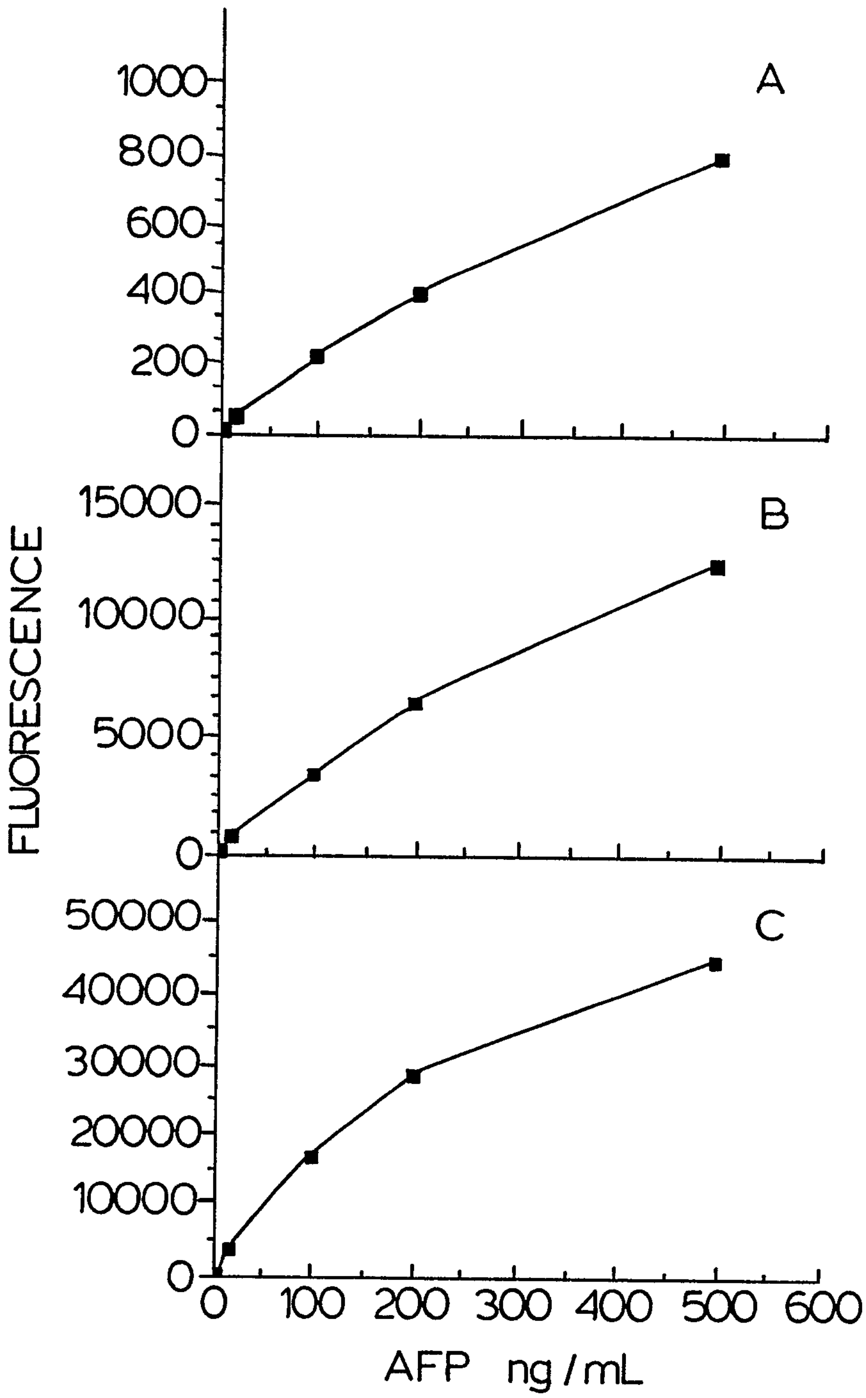
A
B
C
1000
800
600
400
200
0
15000
10000
5000
0
50000
40000
30000
20000
10000
0
0
100
200
300
400
500
600
FLUORESCENCE
AFP ng/mL

FIG. 4

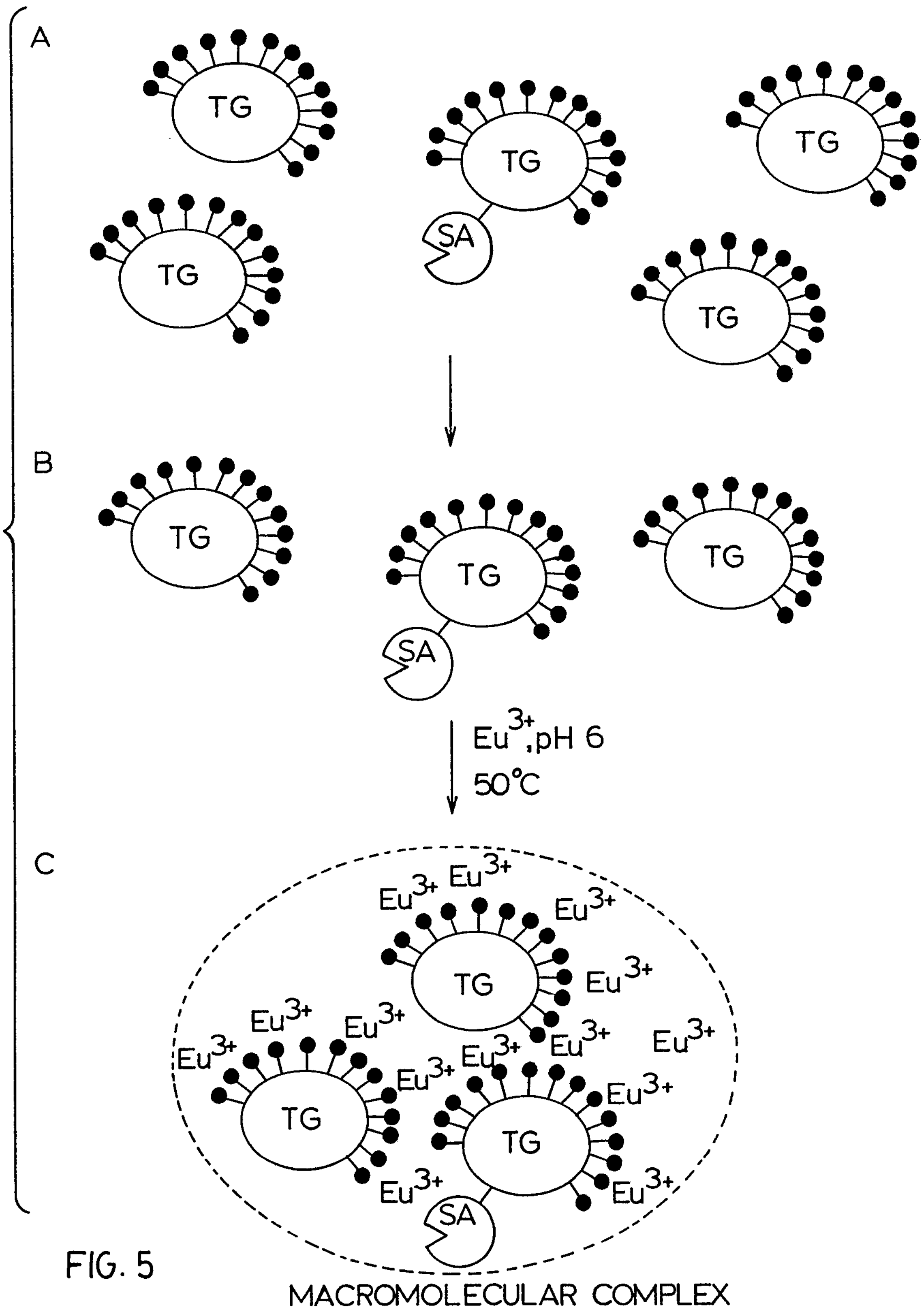


FIG. 5

MACROMOLECULAR COMPLEX

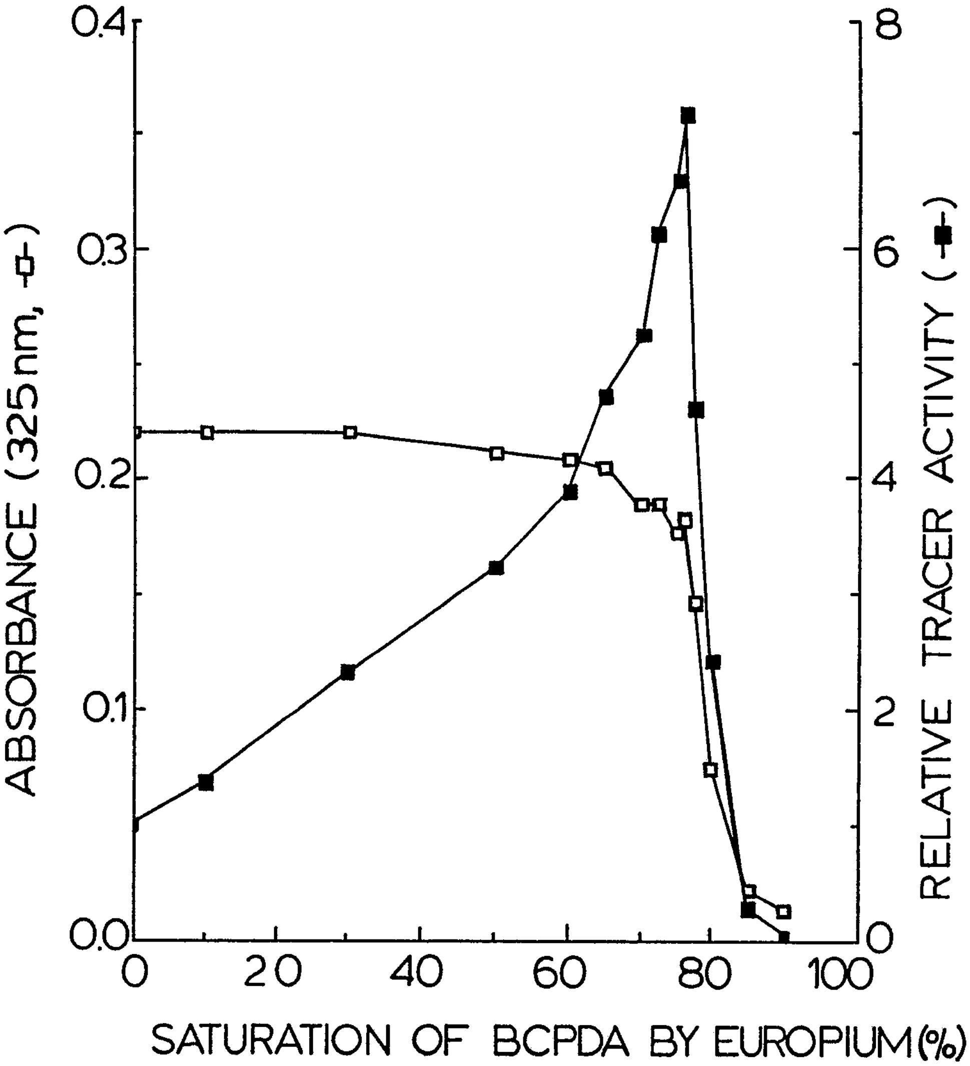
0.4
0.3
0.2
0.1
0.0
ABSORBANCE (325 nm, -□-)
8
6
4
2
0
RELATIVE TRACER ACTIVITY (-■-)
0
20
40
60
80
100
SATURATION OF BCPDA BY EUROPIUM (%)

FIG. 6

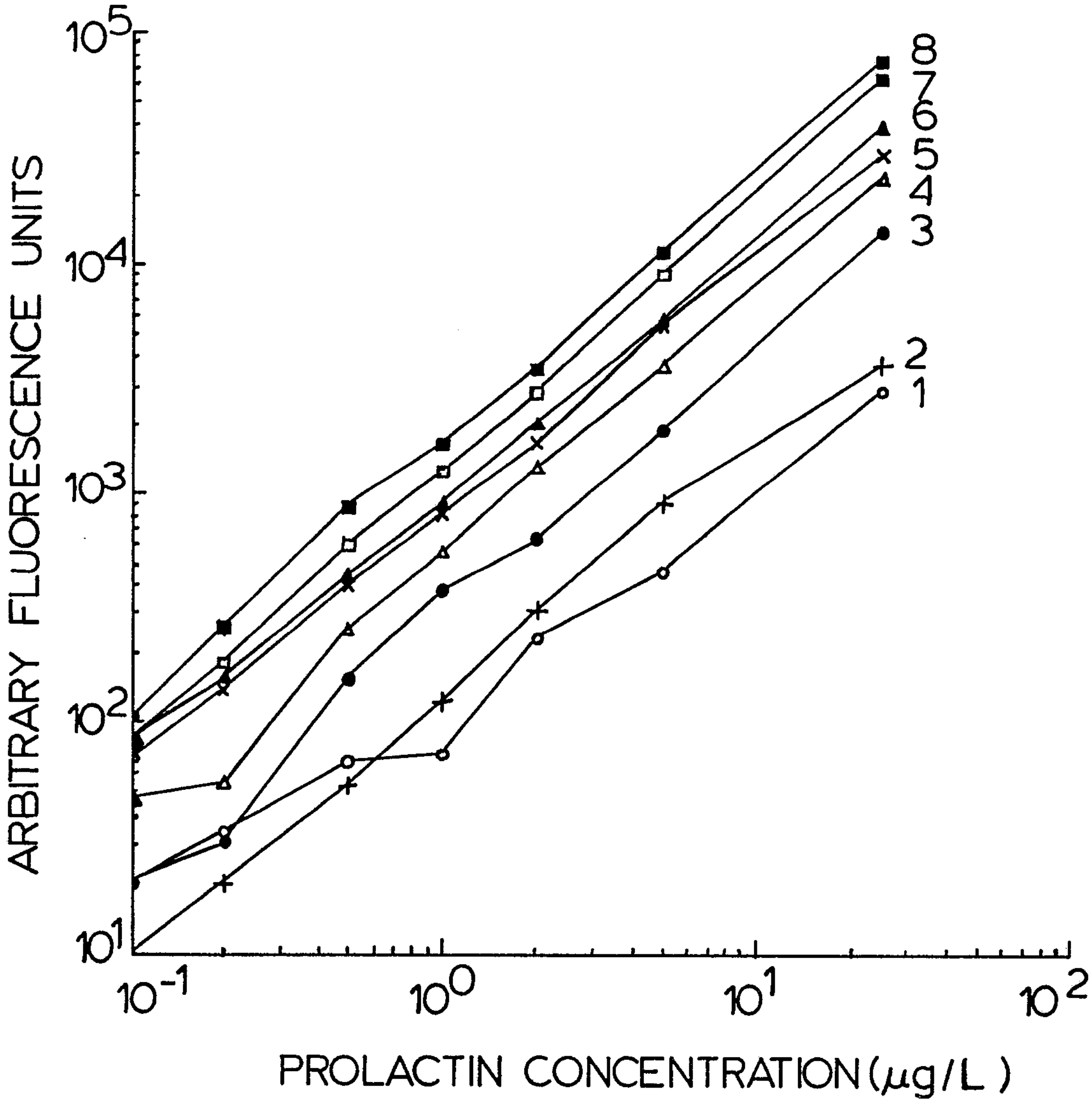
ARBITRARY FLUORESCENCE UNITS
$10^5$
$10^4$
$10^3$
$10^2$
$10^1$
$10^{-1}$
$10^0$
$10^1$
$10^2$
PROLACTIN CONCENTRATION (μg/L)
8
7
6
5
4
3
2
1

FIG. 7

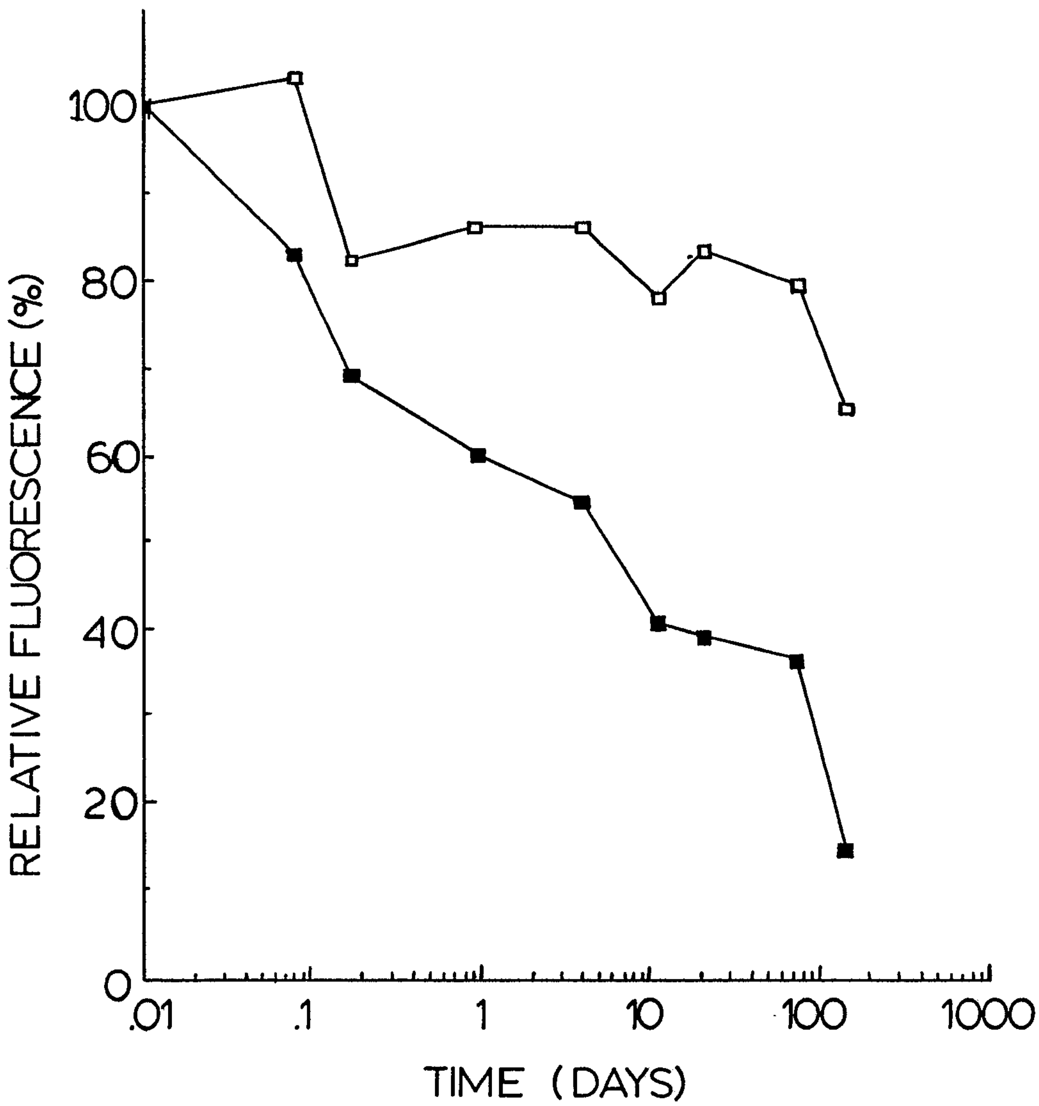
100
80
60
40
20
0
RELATIVE FLUORESCENCE (%)
.01
.1
1
10
100
1000
TIME (DAYS)

FIG. 8

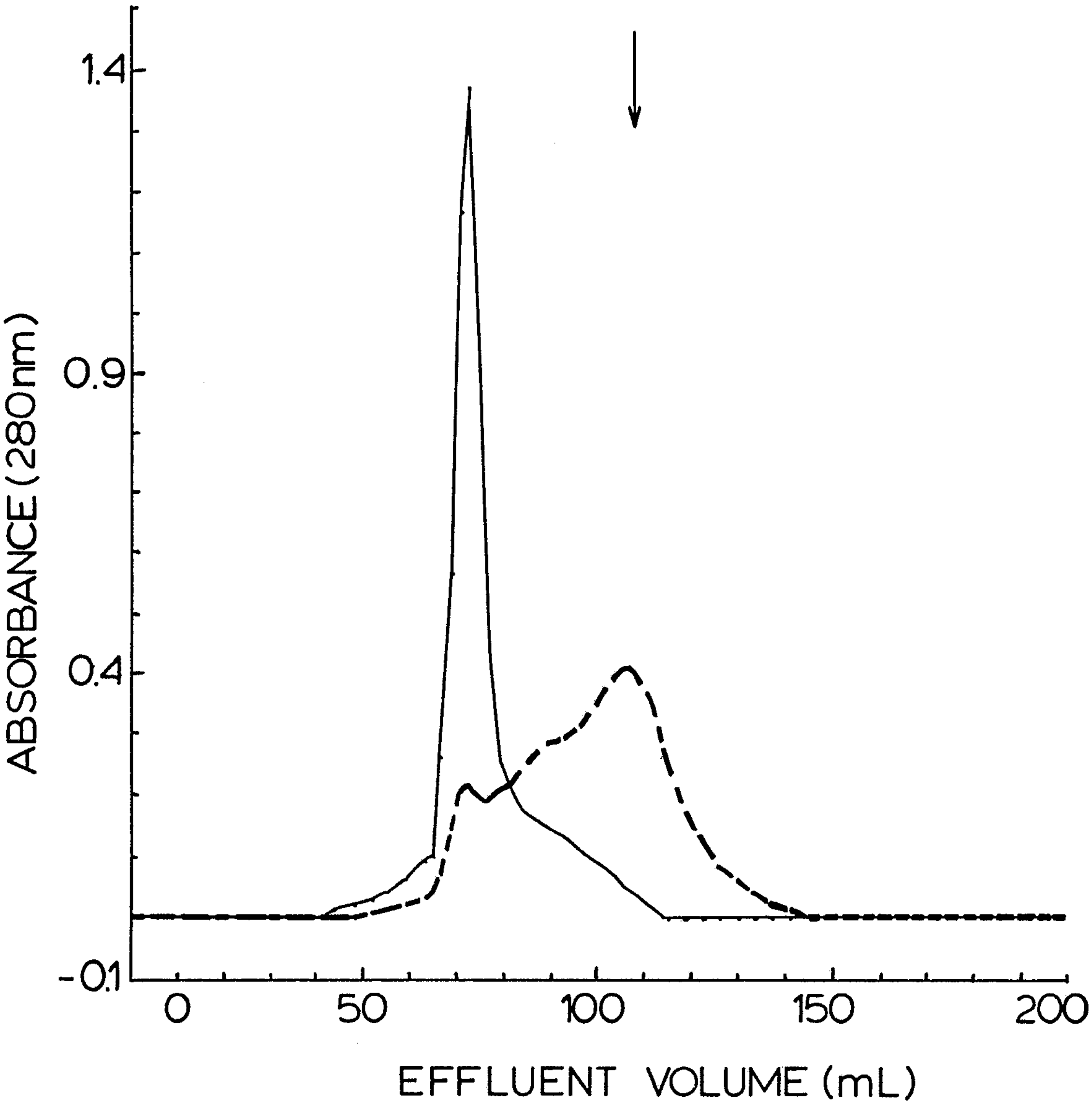
1.4
0.9
0.4
-0.1
ABSORBANCE (280nm)
0
50
100
150
200
EFFLUENT VOLUME (mL)

FIG. 9

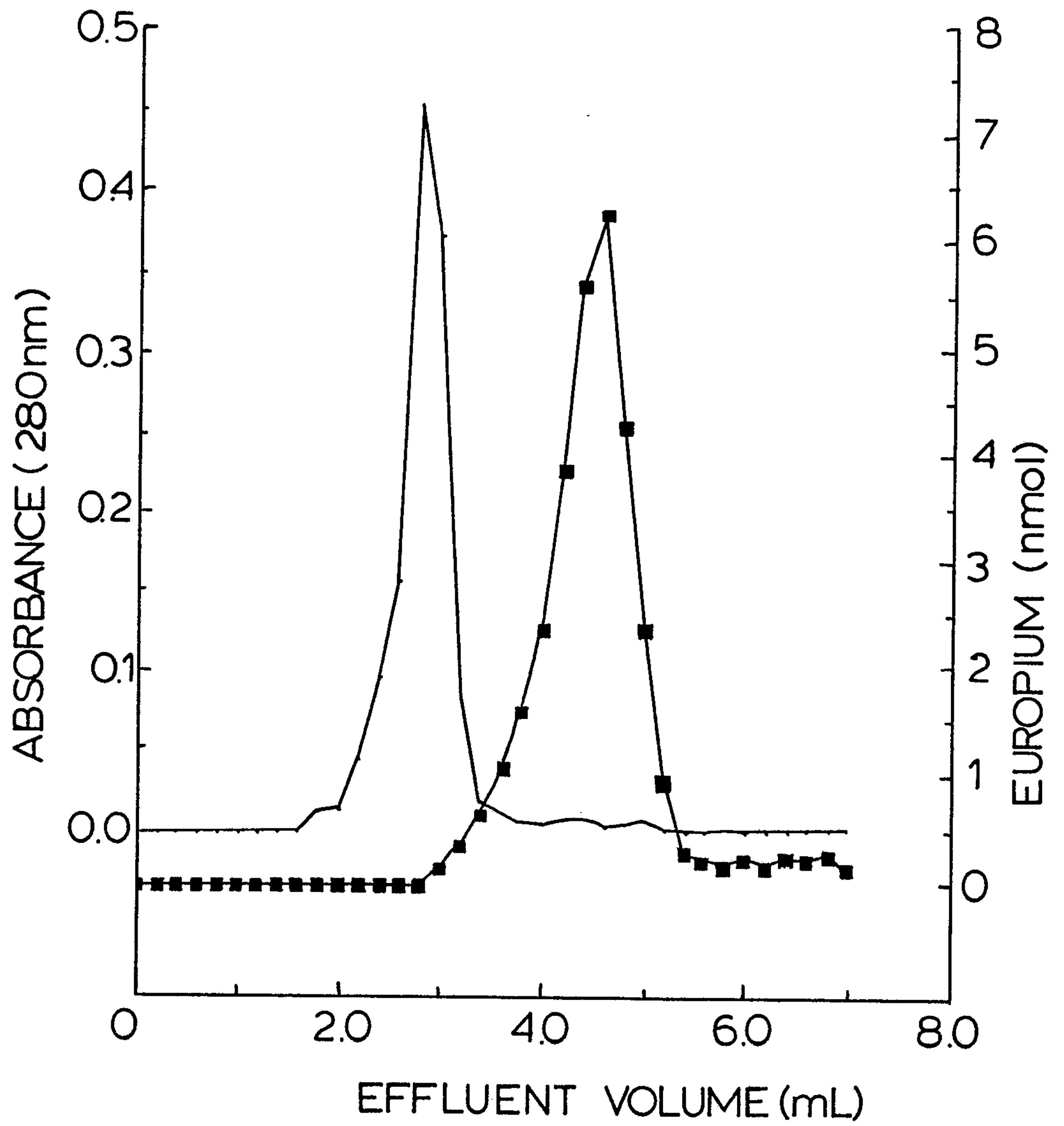
0.5
0.4
0.3
0.2
0.1
0.0
ABSORBANCE ( 280nm)
8
7
6
5
4
3
2
1
0
EUROPIUM (nmol)
0
2.0
4.0
6.0
8.0
EFFLUENT VOLUME (mL)

FIG. 10